(19) Europäisches Patentamt
European Patent Office
Office européen des brevets

(11) **EP 3 978 017 A1**

(12) **EUROPEAN PATENT APPLICATION**
published in accordance with Art. 153(4) EPC

(43) Date of publication:
**06.04.2022 Bulletin 2022/14**

(21) Application number: **20814485.7**

(22) Date of filing: **28.05.2020**

(51) International Patent Classification (IPC):
***A61K 39/395*** *(2006.01)* ***A61K 45/00*** *(2006.01)*
***A61P 43/00*** *(2006.01)* ***A61K 31/4245*** *(2006.01)*

(52) Cooperative Patent Classification (CPC):
**A61K 31/4245; A61K 39/395; A61K 45/00; A61P 43/00**

(86) International application number:
**PCT/JP2020/021108**

(87) International publication number:
**WO 2020/241749 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2019 JP 2019100136**

(71) Applicant: **Taiho Pharmaceutical Co., Ltd.**
**Chiyoda-ku**
**Tokyo 101-8444 (JP)**

(72) Inventors:
- **UENO Hiroyuki**
**Tsukuba-shi, Ibaraki 300-2611 (JP)**
- **TSUKIOKA Sayaka**
**Tsukuba-shi, Ibaraki 300-2611 (JP)**

(74) Representative: **Schiener, Jens**
**Wächtershäuser & Hartz**
**Patentanwaltspartnerschaft mbB**
**Weinstraße 8**
**80333 München (DE)**

(54) **COMBINATION CANCER THERAPY USING SULFONAMIDE COMPOUND AND IMMUNOMODULATORY**

(57) This invention provides a method for enhancing antitumor effects of a ribonucleotide reductase (RNR) inhibitory compound. A pharmaceutical composition for treating and/or preventing a tumor used in combination with an immune checkpoint molecule regulator comprising a sulfonamide compound represented by Formula (I) or a salt thereof is also provided.

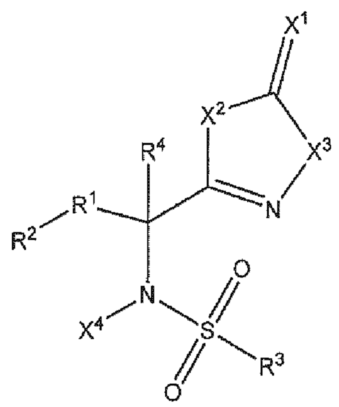

(I)

EP 3 978 017 A1

## Description

### Technical Field

The present disclosure relates to an antitumor agent. More particularly, the present disclosure relates to a pharmaceutical composition comprising a sulfonamide compound or a salt thereof in combination with an immune checkpoint molecule regulator.

### Background Art

Ribonucleotide reductase (hereinafter also referred to as RNR) is composed of a hetero-oligomer of a large subunit M1 and a small subunit M2, and expression of both is required for its enzyme activity. RNR recognizes ribonucleoside 5'-diphosphate (hereinafter also referred to as NDP) as a substrate and catalyzes a reduction reaction to 2'-deoxyribonucleoside 5'-diphosphate (hereinafter also referred to as dNDP). Since RNR is a rate-limiting enzyme in the de novo dNTP synthesis pathway, RNR plays an essential role in DNA synthesis and repair (Non-Patent Literature 1).

The enzymatic activity of RNR is closely related to cell proliferation, and there is a report that the enzymatic activity is particularly high in cancer (Non-Patent Literature 2). Indeed, numerous reports indicate the correlation between the overexpression of M2, one subunit of RNR, and the prognosis of patients in various types of solid tumors and blood cancer (Non-Patent Literature s 3 and 4). In addition, cell growth inhibition and antitumor effect in vivo by inhibiting RNR has been reported in cell lines derived from several cancer types and in nonclinical models (Non-Patent Literature s 5 and 6), strongly suggesting that RNR is an important target molecule for cancer treatment.

Conventionally, hydroxyurea (hereinafter also referred to as HU) and 3-aminopyridine-2-carboxaldehyde thiosemicarbazone (hereinafter also referred to as 3-AP) are known as compounds having an RNR inhibitory activity. These compounds differ in structure from the sulfonamide compounds of the present disclosure. While HU has been used clinically for over 30 years, its RNR inhibitory activity is very weak and its effect is limited (Non-Patent Literature 7). In addition, tolerance to the use of HU is also considered a problem (Non-Patent Literature 8). Meanwhile, 3-AP has a structure which can chelate to metal ions, and it has been known that 3-AP chelates mainly to iron ions, thereby inhibiting RNR (Non-Patent Literature 9). However, 3-AP has been suggested as having an off-target effect to various other iron-ion-requiring proteins, and it has been known that side effects such as hypoxia, dyspnea, methemoglobinemia, and the like are caused in clinical cases (Non-Patent Literature 10).

Therefore, it has been strongly desired to develop an RNR inhibitor which has a better RNR inhibitory activity and a structure which does not chelate with metal ions, and is useful against diseases associated with RNR, such as tumors.

Meanwhile, development of cancer immunotherapy is in progress as a novel method for cancer therapy.

The activation of adaptive immune responses is initiated upon binding of an antigen peptide-MHC complex with a T cell receptor (TCR). Such binding is further defined by costimulation or coinhibition that is caused upon binding between B7 family members (i.e., costimulatory molecules) and CD28 family members (i.e., receptors of costimulatory molecules). In order to activate T cells in an antigen-specific manner, 2 characteristic signal transmission events are necessary, and T cells that had received antigen stimulation only without costimulation by the B7 family become unresponsive (i.e., anergy), and immunological tolerance is induced.

Cancer cells make use of such mechanism and suppress antigen-specific T cell activation. Thus, cancer cells avoid immune surveillance and keep growing. Accordingly, it is considered effective for cancer treatment to enhance costimulation or block coinhibition and induce antitumor immune responses in the body of a cancer patient and regulate tumor immune evasion, and various cancer immunotherapy techniques targeting costimulatory molecules or coinhibitory molecules have been proposed (Non-Patent Literature 11). For example, as an immune checkpoint molecule regulator that inhibits the binding between PD-1 and ligands thereof (PD-L1 and PD-L2) to activate T cells, Nivolumab (human IgG4 monoclonal antibody against human PD-1) is used for the treatment of malignant melanoma (Patent Literature 1 and Non-Patent Literature 12), and Pembrolizumab is used for the treatment of malignant melanoma or non-small cell lung cancer (Non-Patent Literature 12).

The sulfonamide compound represented by Formula (I) shown below or a salt thereof is known as an RNR inhibitor (Patent Literature 2).

However, the RNR inhibitor has not been used in combination with an immune checkpoint molecule regulator.

### Citation List

#### Patent Literature

Patent Literature 1: WO 2004/004771
Patent Literature 2: WO 2017/209155

Non Patent Literature

Non-Patent Literature 1: Annu. Rev. Biochem., 67, 71-98, 1998
Non-Patent Literature 2: J. Biol. Chem., 245, 5228-5233, 1970
Non-Patent Literature 3: Nat. Commun., 5, 3128 doi: 10.1038/ncomms4128, 2014
Non-Patent Literature 4: Clin. Sci., 124, 567-578, 2013
Non-Patent Literature 5: Expert. Opin. Ther. Targets 17, 1423-1437, 2013
Non-Patent Literature 6: Biochem. Pharmacol., 59, 983-991, 2000
Non-Patent Literature 7: Biochem. Pharmacol., 78, 1178-1185, 2009
Non-Patent Literature 8: Cancer Res., 54, 3686-3691, 1994
Non-Patent Literature 9: Pharmacol. Rev., 57, 547-583, 2005
Non-Patent Literature 10: Future Oncol., 8, 145-150, 2012
Non-Patent Literature 11: Nat. Rev. Cancer., 12 (4): 252-64, 2012
Non-Patent Literature 12: N. Engl. J. Med., 366 (26): 2443-54, 2012

Summary of Invention

Technical Problem

The present disclosure provides a method for enhancing antitumor effects achieved with the use of an RNR inhibitory compound.

Solution to Problem

The present inventors have conducted concentrated studies in order to solve the problem described above. As a result, they have discovered that compounds having the sulfonamide structure represented by Formula (I) exert excellent antitumor activity when used in combination with immune checkpoint molecule regulators, which led to the completion of the present disclosure.
The present disclosure provides the following [1] to [16].

[1] A pharmaceutical composition for treating and/or preventing a tumor comprising a sulfonamide compound represented by Formula (I) below or a salt thereof, which is used in combination with an immune checkpoint molecule regulator:

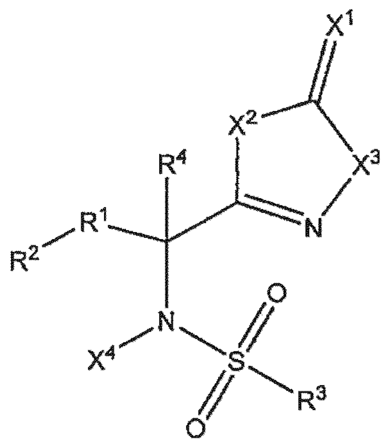
(I)

wherein,

$X^1$ represents an oxygen atom or a sulfur atom;

$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

[2] The pharmaceutical composition according to [1], wherein, in Formula (I):

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
$R^{11}$ and $R^{12}$ are the same or different and represent a hydrogen atom or a C1-C6 alkyl group;
$R^2$ represents a C6-C14 aromatic hydrocarbon group, and $R^2$ may have $R^{21}$ as a substituent;
$R^{21}$ represents a halogen atom or a C1-C6 alkyl group (when 2 or more $R^{21}$ are present, $R^{21}$ may be the same with or different from each other);
$R^3$ represents a C6-C14 aromatic hydrocarbon group which may have $R^{31}$ as a substituent or may be fused with a 4- to 8-membered saturated heterocyclic ring (wherein the saturated heterocyclic ring may have Rc as a substituent);
$R^{31}$ represents a halogen atom or an aminocarbonyl group (when 2 or more $R^{31}$ are present, $R^{31}$ may be the same with or different from each other);
Rc represents a halogen atom, a hydroxy group, or a C1-C6 alkyl group (when 2 or more Rc are present, Rc may be the same with or different from each other); and
$R^4$ represents a hydrogen atom.

[3] The pharmaceutical composition according to [1] or [2], wherein, in Formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
either $R^{11}$ or $R^{12}$ represents a hydrogen atom, and the other represents a C1-C6 alkyl group;
$R^2$ represents a phenyl group, and $R^2$ may have $R^{21}$ as a substituent;
$R^{21}$ represents a halogen atom or a C1-C6 alkyl group (when 2 or more $R^{21}$ are present, $R^{21}$ may be the same with or different from each other);
$R^3$ represents a phenyl group which may have $R^{31}$ as a substituent or may be fused with a monocyclic 6-membered saturated heterocyclic ring having 1 oxygen atom (wherein the saturated heterocyclic ring may have Rc as a substituent);
$R^{31}$ represents a halogen atom or an aminocarbonyl group (when 2 or more $R^{31}$ are present, $R^{31}$ may be the same with or different from each other);

Rc represents a halogen atom, a hydroxy group, or a C1-C6 alkyl group (when 2 or more Rc are present, Rc may be the same with or different from each other); and
$R^4$ represents a hydrogen atom.

[4] The pharmaceutical composition according to any of [1] to [3], wherein, in Formula (I):

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
either $R^{11}$ or $R^{12}$ represents a hydrogen atom, and the other represents a methyl group;
$R^2$ represents a phenyl group having $R^{21}$ as a substituent;
$R^{21}$ represents a halogen atom or a C1-C6 alkyl group (when 2 or more $R^{21}$ are present, $R^{21}$ may be the same with or different from each other);
$R^3$ represents a phenyl group having $R^{31}$ as a substituent or a chromanyl group having Rc as a substituent;
$R^{31}$ represents a halogen atom or an aminocarbonyl group (when 2 or more $R^{31}$ are present, $R^{31}$ may be the same with or different from each other);
Rc represents a halogen atom, a hydroxy group, or a C1-C6 alkyl group (when 2 or more Rc are present, Rc may be the same with or different from each other); and
$R^4$ represents a hydrogen atom.

[5] The pharmaceutical composition according to any of [1] to [4], wherein the sulfonamide compound is 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide.
[6] The pharmaceutical composition according to any of [1] to [5], wherein a sulfonamide compound represented by Formula (I) or a salt thereof and an immune checkpoint molecule regulator are administered concurrently, sequentially, or in a staggered manner.
[7] The pharmaceutical composition according to any of [1] to [6], wherein the immune checkpoint molecule regulator is at least 1 member selected from among a PD-1 pathway antagonist, an ICOS pathway agonist, a CTLA-4 pathway antagonist, and a CD28 pathway agonist.
[8] The pharmaceutical composition according to any of [1] to [7], wherein the immune checkpoint molecule regulator is a PD-1 pathway antagonist.
[9] The pharmaceutical composition according to [8], wherein the PD-1 pathway antagonist is at least 1 member selected from the group consisting of an anti-PD-1 antibody, an anti-PD-Ll antibody, and an anti-PD-L2 antibody.
[10] The pharmaceutical composition according to [8], wherein the PD-1 pathway antagonist is an anti-PD-1 antibody.
[11] The pharmaceutical composition according to [10], wherein the anti-PD-1 antibody is Nivolumab or Pembrolizumab.
[12] A sulfonamide compound represented by Formula (I) or a salt thereof for use in the treatment and/or the prevention of a tumor, which is used in combination with an immune checkpoint molecule regulator:

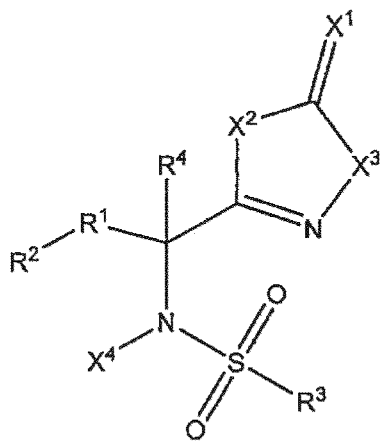

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

[13] Use of a sulfonamide compound represented by Formula (I) or a salt thereof for producing a medicament used for treating and/or preventing a tumor, which is used in combination with an immune checkpoint molecule regulator:

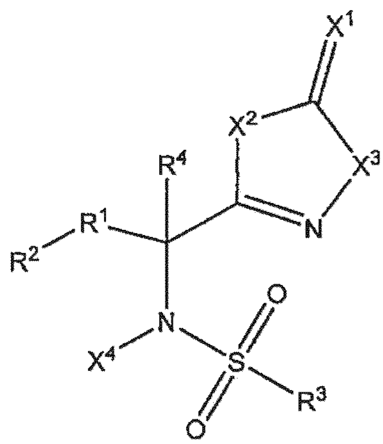
(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic

group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and $R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

[14] A method for treating and/or preventing a tumor comprising administering an effective amount of a sulfonamide compound represented by Formula (I) or a salt thereof to a patient in combination with an immune checkpoint molecule regulator:

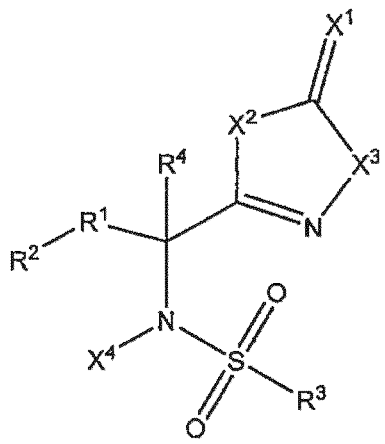

(I)

wherein,

$X^1$ represents an oxygen atom or a sulfur atom;
$x^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

[15] A combination of an immune checkpoint molecule regulator and a sulfonamide compound represented by Formula (I) or a salt thereof used for treating and/or preventing a tumor:

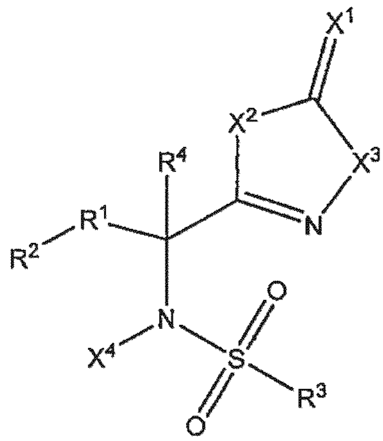

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituteds, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may have substituents; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

[16] An agent for enhancing antitumor effects of an immune checkpoint molecule regulator comprising a sulfonamide compound represented by Formula (I) or a salt thereof:

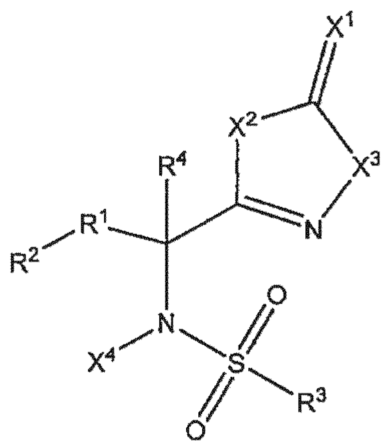

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and $R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

The present disclosure also relates to the following aspects.

- A pharmaceutical composition for preventing and/or treating a tumor comprising a sulfonamide compound represented by Formula (I) or a salt thereof and an immune checkpoint molecule regulator.
- A sulfonamide compound represented by Formula (I) or a salt thereof for enhancing antitumor effects of the immune checkpoint molecule regulator.
- Use of a sulfonamide compound represented by Formula (I) or a salt thereof for enhancing antitumor effects of the immune checkpoint molecule regulator.
- Use of a sulfonamide compound represented by Formula (I) or a salt thereof for manufacturing an agent for enhancing antitumor effects of the immune checkpoint molecule regulator.
- A method of preventing and/or treating a tumor comprising a step of administering prophylactically and/or therapeutically effective amounts of a sulfonamide compound represented by Formula (I) or a salt thereof and the immune checkpoint molecule regulator in combination to a patient.
- A method of preventing and/or treating a tumor comprising a step of administering a prophylactically and/or therapeutically effective amount of a sulfonamide compound represented by Formula (I) or a salt thereof to a cancer patient dosed with the immune checkpoint molecule regulator.
- A method of enhancing an antitumor effect comprising a step of administering a therapeutically and/or prophylactically effective amount of a sulfonamide compound represented by Formula (I) or a salt thereof to a cancer patient dosed

with the immune checkpoint molecule regulator.

- A product comprising a sulfonamide compound represented by Formula (I) or a salt thereof and the immune checkpoint molecule regulator as a combination formulation used concurrently, sequentially, or in a staggered manner in preventing and/or treating a tumor.
- 5-Chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide (hereinafter, may be referred to as "Compound 1") or a salt thereof for use in the treatment and/or the prevention of a tumor, which is used in combination with the immune checkpoint molecule regulator.
- A combination of the immune checkpoint molecule regulator with Compound 1 or a salt thereof for use in the treatment and/or the prevention of a tumor.
- An agent for enhancing antitumor effects of the immune checkpoint molecule regulator comprising Compound 1 or a salt thereof.

The present specification encompasses the contents disclosed in Japanese Patent Application No. 2019-100136, on which the priority of the present application is based. Advantageous Effects of Invention

According to the present disclosure, cancer treatment exerting excellent antitumor effects can be performed while suppressing development of side effects.

Brief Description of Drawings

Fig. 1 shows effects of administration of Compound 1 (25 mg/kg/day) in combination with an anti-mouse PD-1 antibody (0.05 mg/body) on tumor volume changes in mouse models to which MC38 mouse colon cancer cells had been transplanted.

Fig. 2 shows effects of administration of Compound 1 (25 mg/kg/day) in combination with an anti-mouse PD-1 antibody (0.05 mg/body) on body weight changes in mouse models to which MC38 mouse colon cancer cells had been transplanted.

Fig. 3 shows effects of administration of Compound 1 (50 mg/kg/day) in combination with an anti-mouse PD-1 antibody (0.05 mg/body) on tumor volume changes in mouse models to which MC38 mouse colon cancer cells had been transplanted.

Fig. 4 shows effects of administration of Compound 1 (50 mg/kg/day) in combination with an anti-mouse PD-1 antibody (0.05 mg/body) on body weight changes in mouse models to which MC38 mouse colon cancer cells had been transplanted.

Embodiments of the Invention

The present disclosure relates to an antitumor agent, an agent for enhancing antitumor effects, a kit formulation, use of such agents, a method for treating a tumor, and a method for enhancing antitumor effects, characterized by administration of a sulfonamide compound represented by Formula (I) or a salt thereof in combination with an immune checkpoint molecule regulator (an anti-PD-1 antibody, in particular).

In the present disclosure, a sulfonamide compound is represented by Formula (I) below:

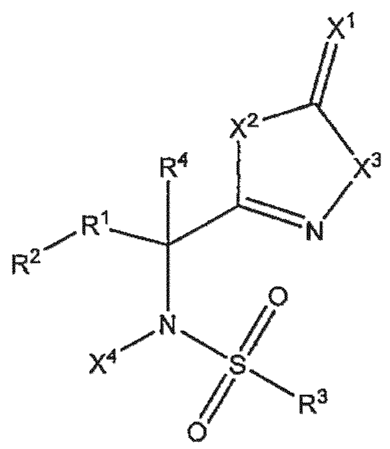

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

"CA-CB" as used herein for description of groups refers to a group having a carbon number of A to B. For example, "C1-C6 alkyl group" represents an alkyl group having 1 to 6 carbon atoms. The term "A- to B-membered" indicates that the number of atoms constituting the ring (ring members) is A to B. For example, "5- to 10-membered unsaturated heterocyclic group" means an unsaturated heterocyclic group whose ring member is 5 to 10.

The "substituent" as used herein includes to a halogen atom, a hydroxy group, an amino group, an oxo group, a cyano group, a nitro group, a carboxyl group, an aminocarbonyl group, a thioamide group, a C1-C6 alkyl group, a C2-C6 alkynyl group, a C3-C6 cycloalkyl group, a C1-C6 alkoxy group, a C1-C6 alkoxy C1-C6 alkoxy group, a halogeno C1-C6 alkyl group, a halogeno C1-C6 alkoxy group, a C6-C14 aromatic hydrocarbon group, an unsaturated heterocyclic group, a saturated heterocyclic group, a nitrogen-containing saturated heterocyclic group, a nitrogen-containing saturated heterocyclic carbonyl group, a C1-C14 acyl group, a C1-C14 acylamino group, a C2-C7 alkoxycarbonyl group, a C1-C14 acyloxy group, C7-C13 aralkyloxy group, and the like.

The "halogen atom" as used herein refers to a fluorine atom, a chlorine atom, a bromine atom, and an iodine atom.

The "C1-C6 alkyl group" as used herein refers to a straight-chain or branched saturated hydrocarbon group having 1 to 6 carbon atoms, such as a methyl group, an ethyl group, an n-propyl group, an isopropyl group, an n-butyl group, an isobutyl group, a tert-butyl group, an n-pentyl group, an isopentyl group, a hexyl group, and the like.

The "C2-C6 alkynyl group" as used herein refers to an unsaturated straight-chain or branched hydrocarbon group having 2 to 6 carbon atoms and at least 1 triple bond, and includes, for example, an ethynyl group, a 1- or 2-propynyl group, a 1-,2- or 3-butynyl group, a 1-methyl-2-propynyl group, and the like.

The "C3-C6 cycloalkyl group" as used herein refers to a saturated cyclic hydrocarbon group having 3 to 6 carbon atoms, and includes, for example, a cyclopropyl group, a cyclobutyl group, a cyclopentyl group, a cyclohexyl group, and the like.

The "C1-C6 alkoxy group" as used herein refers to an oxy group to which a straight-chain or branched saturated hydrocarbon group having 1 to 6 carbon atoms binds, and includes, for example, a methoxy group, an ethoxy group, a propoxy group, an isopropoxy group, an n-butoxy group, an isobutoxy group, a tert-butoxy group, a pentyloxy group, an isopentyloxy group, a hexyloxy group, and the like.

The "C1-C6 alkoxy C1-C6 alkoxy group" as used herein refers to a C1-C6 alkoxy group in which one hydrogen atom of the C1-C6 alkoxy group is substituted with a C1-C6 alkoxy group, and includes, for example, a methoxymethoxy group, a methoxyethoxy group, a methoxypropoxy group, an ethoxymethoxy group, an ethoxyethoxy group, a propoxy methoxy group, and the like.

The "halogeno C1-C6 alkyl group" as used herein refers to a C1-C6 alkyl group in which one or more hydrogen atoms are substituted with a halogen atom, and includes, for example, a fluoromethyl group, a difluoromethyl group, a trifluoromethyl group, a trichloromethyl group, a fluoroethyl group, a 1,1,1-trifluoroethyl group, a monofluoro-n-propyl group, a perfluoro-n-propyl group, a perfluoroisopropyl group, and the like.

The "C6-C14 aromatic hydrocarbon group" as used herein refers to a monocyclic or polycyclic aromatic hydrocarbon group having 6 to 14 carbon atoms, and includes, for example, a phenyl group, a naphthyl group, an anthracenyl

group, a phenanthryl group, a fluorenyl group, and the like.

The "unsaturated heterocyclic group" as used herein refers to a monocyclic or polycyclic unsaturated heterocyclic group having at least 1 (preferably 1 to 4, and more preferably 1 to 3) heteroatom selected from among a nitrogen atom, a sulfur atom, and an oxygen atom. The unsaturated heterocyclic group includes a fully unsaturated heterocyclic group and a partially unsaturated heterocyclic group.

A fully unsaturated heterocyclic group includes, for example, a pyrrolyl group, an imidazolyl group, a pyrazolyl group, a triazolyl group, a tetrazolyl group, a furanyl group (a furyl group), an oxazolyl group, an isoxazolyl group, an oxadiazolyl group, a thiophenyl group (a thienyl group), a thiazolyl group, an isothiazolyl group, a thiadiazolyl group, a pyridinyl group (a pyridyl group), a pyrimidinyl group (a pyrimidyl group), a pyrazinyl group (a pyrazyl group), a pyridazinyl group, an indolyl group, an isoindolyl group, an indazolyl group (a benzpyrazol group), a benzimidazolyl group, a benzotriazolyl group, an azaindolyl group, a pyrrolopyridinyl group, an imidazopyridinyl group, a pyrazolopyridinyl group, a triazolopyridinyl group, a pyrrolopyrimidinyl group, an imidazopyrimidinyl group, a pyrazolopyrimidinyl group, a benzofuranyl group, a benzoxazolyl group, a benzothiophenyl group (a benzothienyl group), a benzothiazolyl group, a benzothiadiazolyl group, a benzofuranyl group (a benzofuryl group), a quinolyl group, an isoquinolyl group, a quinazolinyl group, a quinoxalyl group, and the like.

A partially unsaturated heterocyclic group includes, for example, a dihydropyranyl group, a dihydrotriazolyl group, a dihydrofuranyl group, a dihydrooxadiazolyl group, a dihydroquinolyl group, a dihydroquinazolinyl group, an indolinyl group, a tetrahydroisoquinolyl group, a methylenedioxyphenyl group, an ethylenedioxyphenyl group, a dihydrobenzofuranyl group, a dihydrobenzoxazolyl group, a dihydropyridooxazinyl group, and the like.

The "saturated heterocyclic group" as used herein refers to a monocyclic or polycyclic fully saturated heterocyclic group having at least 1 (preferably 1 to 4, and more preferably 1 to 3) heteroatom selected from among a nitrogen atom, a sulfur atom, and an oxygen atom. Specific examples include an azetidinyl group, a pyrrolidinyl group, a piperidinyl group, a piperazinyl group, a hexamethyleneimino group, a morpholino group, a thiomorpholino group, a homopiperazinyl group, a tetrahydrofuranyl group, a tetrahydropyranyl group, a tetrahydrothiophenyl group, a thiazolidinyl group, and an oxazolidinyl group.

The "nitrogen-containing saturated heterocyclic group" as used herein refers to a saturated heterocyclic group having one or more nitrogen atoms and optionally having a heteroatom other than a nitrogen atom, and include, for example, a morpholino group.

The "nitrogen-containing saturated heterocyclic carbonyl group" as used herein refers to a carbonyl group to which a nitrogen-containing saturated heterocyclic group binds, and includes, for example, a morpholinocarbonyl group.

The "C1-C14 acyl group" as used herein refers to a carbonyl group to which a hydrogen atom, a C1-C6 alkyl group, a C6-C14 aromatic hydrocarbon group, or an unsaturated heterocyclic group binds, and examples thereof include: a formyl group; a (C1-C6 alkyl) carbonyl group such as an acetyl group, a propanoyl group, and a butanoyl group; a (C3-C6 cycloalkyl) carbonyl group such as a cyclopropanoyl group and a cyclobutanoyl group; and a (C6-C13) arylcarbonyl group such as a benzoyl group, a naphthylcarbonyl group, and a fluorenylcarbonyl group.

The "C1-C14 acylamino group" as used herein refers to an amino group in which 1 or 2 hydrogen atoms are substituted with a C1-C14 acyl group, and examples thereof include an acetylamino group, a propanoylamino group, a butanoylamino group, and a cyclopropanoyl amino group.

The "C2-C7 alkoxycarbonyl group" as used herein refers to a carbonyl group to which a C1-C6 alkoxy group binds, and examples thereof include a methoxycarbonyl group, an ethoxycarbonyl group, an n-propoxycarbonyl group, an isopropoxycarbonyl group, an n-butoxycarbonyl group, and a tert-butoxycarbonyl group.

The "C1-C14 acyloxy group" as used herein includes, for example, a formyloxy group; a (C1-C6 alkyl)carbonyloxy group such as a methyl carbonyloxy group, an ethyl carbonyloxy group, an n-propyl carbonyloxy group, an isopropylcarbonyloxy group, an n-butylcarbonyloxy group, an isobutylcarbonyloxy group, a tert-butylcarbonyloxy group, an n-pentylcarbonyloxy group, an isopentylcarbonyloxy group, and a hexylcarbonyloxy group,; a (C3-C6 cycloalkyl)carbonyloxy group such as a cyclopropanoyloxy group, and a cyclobutanoyloxy group; and a (C6-C13 aryl)carbonyloxy group such as a phenylcarbonyloxy group, a naphthylcarbonyloxy group, and a fluorenylcarbonyloxy group.

The "C7-C13 aralkyloxy group" as used herein refers to an alkyloxy group in which one hydrogen atom is substituted with an aryl group, and examples thereof include a benzyloxy group, a phenethyloxy group, a naphthylmethyloxy group, and a fluorenylmethyloxy group.

The "saturated or partially unsaturated hydrocarbon ring" as used herein refers to a monocyclic or polycyclic saturated or partially unsaturated hydrocarbon ring, and examples thereof include a cyclopropane ring, a cyclobutane ring, a cyclopentane ring, a cyclohexane ring, a cycloheptane ring, a cyclooctane ring, a cyclobutene ring, a cyclopentene ring, a cyclohexene ring, a cycloheptene ring, and a cyclooctadiene ring.

The "saturated or partially unsaturated heterocyclic ring" as used herein refers to a monocyclic or polycyclic saturated or partially unsaturated heterocyclic ring having a heteroatom selected from among a nitrogen atom, a sulfur atom, and an oxygen atom, and examples thereof include an oxirane ring, an azetidine ring, a pyrrolidine ring, an imidazolidine ring, a piperidine ring, a piperazine ring, a morpholine ring, a tetrahydrofuran ring, a tetrahydropyran ring,

a dioxane ring, a tetrahydrothiophene ring, a dihydropyran ring, and a dihydrofuran ring.

The "spiroheterocyclic group" as used herein refers to a saturated or unsaturated spiroheterocyclic group having a heteroatom selected from among a nitrogen atom, a sulfur atom, and an oxygen atom and a spirocarbon atom, and examples thereof include a 2-oxa-6-azaspiro[3.4]octanyl group and a 2-oxa-7-azaspiro[3.5]nonanyl group.

The "bridged heterocyclic group" as used herein refers to a bicyclic or higher bridged heterocyclic group, which has a heteroatom selected from among a nitrogen atom, a sulfur atom, and an oxygen atom and 2 bridgehead carbons, and examples thereof include a 3-oxa-8-azabicyclo[3.2.1]octanyl group and an 8-oxa-3-azabicyclo[3.2.1]octanyl group.

In the compounds represented by Formula (I) herein, $X^1$ is an oxygen atom or a sulfur atom. $X^1$ is preferably an oxygen atom.

In the compounds represented by Formula (I) herein, $X^2$ is an oxygen atom or -NH-. $X^2$ is preferably an oxygen atom.

In the compounds represented by Formula (I) herein, $X^3$ is -NH- or an oxygen atom. $X^3$ is preferably -NH-.

In the compounds represented by Formula (I), $X^4$ is a hydrogen atom or a C1-C6 alkyl group.

The "C1-C6 alkyl group" represented by $X^4$ is preferably a C1-C3 alkyl group, and more preferably a methyl group.

$X^4$ is preferably a hydrogen atom or a methyl group, and more preferably a hydrogen atom.

In the compounds represented by Formula (I), $R^1$ is $-C(R^{11})(R^{12})-$ or $-C(=CH_2)-$.

In $-C(R^{11})(R^{12})-$, $R^{11}$ and $R^{12}$ are the same or different and are a hydrogen atom, a halogen atom, a hydroxy group, or a C1-C6 alkyl group. Alternatively, $R^{11}$ and $R^{12}$ form, together with the carbon atoms to which they bind, a saturated hydrocarbon ring having 3 to 8 carbon atoms.

The "halogen atom" represented by $R^{11}$ and $R^{12}$ is preferably a fluorine atom, a chlorine atom, or a bromine atom, and more preferably a fluorine atom.

The "C1-C6 alkyl group" represented by $R^{11}$ and $R^{12}$ is preferably a C1-C3 alkyl group, more preferably a methyl group or an ethyl group, and further preferably a methyl group.

The "saturated hydrocarbon ring having 3 to 8 carbon atoms," which is formed by $R^{11}$ and $R^{12}$ together with the carbon atoms to which they bind, is preferably a monocyclic saturated hydrocarbon ring having 3 to 6 carbon atoms, and more preferably a cyclopropane ring.

Preferably, $R^{11}$ is a halogen atom, a hydroxy group, or a C1-C6 alkyl group, and $R^{12}$ is a hydrogen atom, a halogen atom, a hydroxy group, or a C1-C6 alkyl group. Alternatively, $R^{11}$ and $R^{12}$ form, together with the carbon atoms to which they bind, a saturated hydrocarbon ring having 3 to 8 carbon atoms. More preferably, $R^{11}$ is a C1-C6 alkyl group, and $R^{12}$ is a hydrogen atom. Particularly preferably, $R^{11}$ is a methyl group, and $R^{12}$ is a hydrogen atom.

$R^1$ is preferably $-C(R^{11})(R^{12})-$, in which $R^{11}$ is a halogen atom, a hydroxy group, or a C1-C6 alkyl group, and $R^{12}$ is a hydrogen atom, a halogen atom, a hydroxy group, or a C1-C6 alkyl group. Alternatively, $R^{11}$ and $R^{12}$ form, together with the carbon atoms to which they bind, a saturated hydrocarbon ring having 3 to 8 carbon atoms. More preferably, $R^1$ is $-C(R^{11}) (R^{12})-$, in which $R^{11}$ is a C1-C6 alkyl group, and $R^{12}$ is a hydrogen atom. Further preferably, $R^1$is $-CH(CH_3)-$.

In the compounds represented by Formula (I), $R^2$ is a C6-C14 aromatic hydrocarbon group or a 9- to 10-membered fully unsaturated heterocyclic group.

The "C6-C14 aromatic hydrocarbon group" represented by $R^2$ is preferably a C6-C10 aromatic hydrocarbon group, more preferably a phenyl group or a naphthyl group, and particularly preferably a phenyl group.

Furthermore, the "9- to 10-membered fully unsaturated heterocyclic group" represented by $R^2$ is preferably a bicyclic 9- to 10-membered fully unsaturated heterocyclic group having 1 to 3 heteroatoms selected from among a nitrogen atom, a sulfur atom, and an oxygen atom, more preferably a bicyclic 9- to 10-membered fully unsaturated heterocyclic group having 1 to 2 heteroatoms selected from a nitrogen atom and a sulfur atom, even more preferably a benzothiophenyl group, a benzothiazolyl group, or a quinolyl group.

In the compounds represented by Formula (I), $R^2$ may be unsubstituted or substituted. When $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or a heterocyclic ring which may be substituted.

When $R^2$ has substituent(s), a position of substitution is not particularly limited. When $R^2$ is a phenyl group, for example, a position of substitution is preferably at position 2, 3, 5, or 6. While the number of substituent is not particularly limited, it is preferably 0, i.e., unsubstituted, or 1 to 4, and it is more preferably 1 to 4 or 1 to 3. When the number of substituents is 2 or more, the types of substituents may be the same with or different from each other.

In the compounds represented by Formula (I), $R^2$ may be preferably substituted with the above "substituent," and more preferably $R^2$ may be substituted with $R^{21}$. When $R^2$ has 2 substituents on the carbon atoms adjacent to each other on the aromatic hydrocarbon ring, the substituents may be preferably fused together with the carbon atoms to which they bind to form a 4- to 8-membered saturated or partially unsaturated hydrocarbon ring or heterocyclic ring which may be substituted with Rz.

$R^{21}$, which can bind to $R^2$ as a substituent, is a halogen atom, an aminocarbonyl group, a cyano group, a C1-C6 alkyl group which may be substituted with Rx, a C3-C6 cycloalkyl group which may be substituted with Rx, a C2-C6 alkynyl group which may be substituted with Rx, a C6-C14 aromatic hydrocarbon group which may be substituted with Ry, or a 5- to 10-membered unsaturated heterocyclic ring which may be substituted with Rz.

The position of substitution by $R^{21}$ is not particularly limited. When $R^2$ is a phenyl group, for example, it is preferably at position 2, 3, 5, or 6. While the number of the substituent $R^{21}$ is not particularly limited, it is preferably 0, i.e., unsubstituted, or 1-4, and it is more preferably 1 to 4 or 1 to 3. When the number of the substituent $R^{21}$ is 2 or more, the types of substituents may be the same with or different from each other.

The "halogen atom" represented by $R^{21}$ is preferably a fluorine atom, a chlorine atom, or a bromine atom.

The "C1-C6 alkyl group" in the "C1-C6 alkyl group which may be substituted with Rx" represented by $R^{21}$ is preferably a C1-C3 alkyl group, and more preferably a methyl group or an ethyl group.

The substituent Rx in the "C1-C6 alkyl group which may be substituted with Rx" represented by $R^{21}$ is a halogen atom or a C6-C14 aromatic hydrocarbon group. The substituent Rx is preferably a halogen atom, and more preferably a fluorine atom. While the number of Rx to bind to a C1-C6 alkyl group as a substituent is not particularly limited, it is preferably 0, i.e., unsubstituted, or 1-3. When the number of substituent Rx is 2 or more, the types of substituents may be the same with or different from each other.

The "C3-C6 cycloalkyl group" in the "C3-C6 cycloalkyl group which may be substituted with Rx" represented by $R^{21}$ is preferably a cyclopropyl group.

The substituent Rx in the "C3-C6 cycloalkyl group which may be substituted with Rx" represented by $R^{21}$ is, as described above, a halogen atom or a C6-C14 aromatic hydrocarbon group, preferably a halogen atom, and more preferably a fluorine atom. While the number of Rx to bind to a C3-C6 cycloalkyl group as a substituent is not particularly limited, it is preferably 0, i.e., unsubstituted, or 1, and it is more preferably 0. When the number of substituent Rx is 2 or more, the types of substituents may be the same with or different from each other.

The "C2-C6 alkynyl group" in the "C2-C6 alkynyl group which may be substituted with Rx" represented by $R^{21}$ is preferably a C2-C4 alkynyl group, and more preferably an ethynyl group.

The substituent Rx in the "C2-C6 alkynyl group which may be substituted with Rx" represented by $R^{21}$ is, as described above, a halogen atom or a C6-C14 aromatic hydrocarbon group, preferably a C6-C14 aromatic hydrocarbon group, more preferably a C6-C10 aromatic hydrocarbon group, and more preferably a phenyl group. While the number of Rx to bind to a C2-C6 alkynyl group as a substituent is not particularly limited, it is preferably 0, i.e., unsubstituted, or 1, and it is more preferably 1. When the number of substituent Rx is 2 or more, the types of substituents may be the same with or different from each other.

The "C6-C14 aromatic hydrocarbon group" in the "C6-C14 aromatic hydrocarbon group which may be substituted with Ry" represented by $R^{21}$ is preferably a C6-C10 aromatic hydrocarbon group, and more preferably a phenyl group.

The substituent Ry in the "C6-C14 aromatic hydrocarbon group which may be substituted with Ry" represented by $R^{21}$ is a halogen atom or a C1-C6 alkoxy group.

The halogen atom represented by Ry is preferably a fluorine atom or chlorine atom. The C1-C6 alkoxy group represented by Ry is preferably a C1-C3 alkoxy group, and more preferably a methoxy group. The substituent Ry in the "C6-C14 aromatic hydrocarbon group which may be substituted with Ry" represented by $R^{21}$ is preferably a fluorine atom, a chlorine atom, or a C1-C3 alkoxy group, and more preferably a fluorine atom, a chlorine atom, or a methoxy group. While the number of Ry to bind to a C6-C14 aromatic hydrocarbon group as a substituent is not particularly limited, it is preferably 0, i.e., unsubstituted, or 1 or 2. When the number of the substituent Ry is 2 or more, the types of substituents may be the same with or different from each other.

The "5- to 10-membered unsaturated heterocyclic group" in the "5- to 10-membered unsaturated heterocyclic group which may be substituted with Rz" represented by $R^{21}$ is preferably a monocyclic or bicyclic 5- to 10-membered fully or partially unsaturated heterocyclic group having 1 to 3 heteroatoms selected from among a nitrogen atom, a sulfur atom, and an oxygen atom, more preferably a monocyclic or bicyclic 5- to 10-membered unsaturated heterocyclic group having 1 to 3 heteroatoms selected from among a nitrogen atom, a sulfur atom, and an oxygen atom, and further preferably a monocyclic 5- to 6-membered unsaturated heterocyclic group having 1 to 3 nitrogen or oxygen atoms. It is preferably a pyrrolyl group, an imidazolyl group, a pyrazolyl group, a pyridyl group, a pyrimidyl group, an oxazolyl group, or a dihydropyridooxazinyl group, more preferably a pyrazolyl group, a pyridyl group, a pyrimidyl group, an oxazolyl group, or a dihydropyridooxazinyl group, and further preferably a pyrazolyl group.

The substituent Rz in the "5- to 10-membered unsaturated heterocyclic group which may be substituted with Rz" represented by $R^{21}$ is a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, or a nitrogen-containing saturated heterocyclic carbonyl group.

The "halogen atom" represented by Rz is preferably a fluorine atom or a chlorine atom.

The "C1-C6 alkyl group" represented by Rz is preferably a C1-C3 alkyl group, and more preferably a methyl

group or an ethyl group.

The "halogeno C1-C6 alkyl group" represented by Rz is preferably a halogeno C1-C3 alkyl group, and more preferably a difluoromethyl group or a trifluoromethyl group.

The "C3-C6 cycloalkyl group" represented by Rz is preferably a cyclopropyl group or a cyclobutyl group.

The "C1-C6 alkoxy group" represented by Rz is preferably a C1-C3 alkoxy group, and more preferably a methoxy group.

The "C6-C14 aromatic hydrocarbon group" represented by Rz is preferably a phenyl group.

The "nitrogen-containing saturated heterocyclic group" represented by Rz is preferably a morpholino group or a piperidinyl group.

The "nitrogen-containing saturated heterocyclic carbonyl group" represented by Rz is preferably a morpholinocarbonyl group.

The substituent Rz in the "5- to 10-membered unsaturated heterocyclic group which may be substituted with Rz" is preferably a halogen atom, a C1-C6 alkyl group, a halogeno C1-C6 alkyl group, a C3-C6 cycloalkyl group, a C1-C6 alkoxy group, a phenyl group, a morpholino group, a piperidinyl group, or a morpholinocarbonyl group, more preferably a C1-C6 alkyl group, and more preferably a methyl group. While the number of Rz which binds to the 5- to 10-membered unsaturated heterocyclic group as a substituent is not particularly limited, it is preferably 0, i.e., unsubstituted, or preferably 1 or 2. When the number of the substituent Rz is 2 or more, the types of substituents may be the same with or different from each other.

$R^{21}$, which can bind to $R^2$ as a substituent, is preferably a halogen atom, an aminocarbonyl group, a cyano group, a C1-C6 alkyl group (which may be substituted with a halogen atom), a C3-C6 cycloalkyl group, a C2-C6 alkynyl group (which may be substituted with a C6-C14 aromatic hydrocarbon group), a C6-C14 aromatic hydrocarbon group (which may be substituted with a group selected from the group consisting of a halogen atom and a C1-C6 alkoxy group), or a monocyclic or bicyclic 5- to 10-membered unsaturated heterocyclic group having 1 to 3 heteroatoms selected from among a nitrogen atom, a sulfur atom, and an oxygen atom (which may be substituted with a group selected from the group consisting of a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, and a nitrogen-containing saturated heterocyclic carbonyl group).

More preferably, $R^{21}$ is a halogen atom, a cyano group, a C1-C6 alkyl group (which may be substituted with a halogen atom), a C3-C6 cycloalkyl group, a phenyl group (which may be substituted with a group selected from the group consisting of a halogen atom and a C1-C6 alkoxy group), or a monocyclic or bicyclic 5- to 10-membered unsaturated heterocyclic group having 1 to 3 heteroatoms selected from among a nitrogen atom, a sulfur atom, and an oxygen atom (which may be substituted with a group selected from the group consisting of a halogen atom, a C1-C6 alkyl group, a halogeno C1-C6 alkyl group, a C3-C6 cycloalkyl group, a C1-C6 alkoxy group, a morpholino group, a piperidinyl group, and a morpholinocarbonyl group).

More preferably, $R^{21}$ is a halogen atom, a C1-C6 alkyl group, or a monocyclic 5- or 6-membered unsaturated heterocyclic group having 1 to 3 nitrogen atoms (which may be substituted with a C1-C6 alkyl group).

More preferably, $R^{21}$ is a halogen atom or a C1-C6 alkyl group.

In the compounds represented by Formula (I), when the number of the substituents for $R^2$ is 2 or more and $R^2$ has 2 substituents on the carbon atoms adjacent to each other on the aromatic hydrocarbon ring, the "4- to 8-membered saturated or partially unsaturated hydrocarbon ring or heterocyclic ring which may be substituted" formed by the substituents together with the carbon atoms to which they bind is a ring fused to the aromatic hydrocarbon ring, for example, a benzene ring. The "4- to 8-membered saturated or partially unsaturated hydrocarbon ring or heterocyclic ring" in the "4- to 8-membered saturated or partially unsaturated hydrocarbon ring or heterocyclic ring which may be substituted" is preferably a monocyclic saturated or partially unsaturated hydrocarbon ring having 4 to 8 carbon atoms or a monocyclic 4- to 8-membered saturated or partially unsaturated heterocyclic ring having 1 to 3 heteroatoms selected from among a nitrogen atom, a sulfur atom, and an oxygen atom, more preferably a monocyclic saturated or partially unsaturated hydrocarbon ring having 4 to 8 carbon atoms, more preferably a monocyclic saturated or partially unsaturated hydrocarbon ring having 4 to 6 carbon atoms or a monocyclic 4- to 6-membered saturated or partially unsaturated heterocyclic ring having 1 to 3 heteroatoms selected from among a nitrogen atom, a sulfur atom, and an oxygen atom, more preferably a monocyclic saturated or partially unsaturated hydrocarbon ring having 5 or 6 carbon atoms, and more preferably a monocyclic saturated hydrocarbon ring having 5 carbon atoms.

The substituent Rz in the the "4- to 8-membered saturated or partially unsaturated hydrocarbon ring or heterocyclic ring which may be substituted with Rz" is, as described above, a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, or a nitrogen-containing saturated heterocyclic carbonyl group, preferably a C1-C6 alkyl group, more preferably a C1-C3 alkyl group, and even more preferably a methyl group. While the number of Rz to bind to a saturated or partially unsaturated hydrocarbon ring or heterocyclic ring as a substituent is not particularly limited, it is preferably 0, i.e., unsubstituted, or 1, and it is more preferably 0, i.e., unsubstituted. When the number of

the substituent Rz is 2 or more, the types of substituents may be the same with or different from each other.

The "4- to 8-membered saturated or partially unsaturated hydrocarbon ring or heterocyclic ring which may be substituted with Rz" is preferably a monocyclic saturated or partially unsaturated hydrocarbon ring having 4 to 8 carbon atoms, which may be substituted with Rz, or a monocyclic 4- to 8-membered saturated or partially unsaturated heterocyclic ring having 1 to 3 heteroatoms selected from among a nitrogen atom, a sulfur atom, and an oxygen atom, more preferably a monocyclic saturated or partially unsaturated hydrocarbon ring having 4 to 8 carbon atoms (which may be substituted with a C1-C6 alkyl group) or a monocyclic 4-to 8-membered saturated or partially unsaturated heterocyclic ring having 1 to 3 heteroatoms selected from among a nitrogen atom, a sulfur atom, and an oxygen atom (which may be substituted with a C1-C6 alkyl group), more preferably a monocyclic saturated or partially unsaturated hydrocarbon ring having 4 to 8 carbon atoms (which may be substituted with a C1-C6 alkyl group), and more preferably a monocyclic saturated or partially unsaturated hydrocarbon ring having 5 or 6 carbon atoms (which may be substituted with a C1-C6 alkyl group).

In the compounds represented by Formula (I), the fused ring, which is formed when the compound has 2 substituents on the carbon atoms adjacent to each other on the aromatic hydrocarbon ring of $R^2$, is for example, a dihydroindene ring, a tetrahydronaphthalene ring, or a dihydrobenzofuran ring.

In the compounds represented by Formula (I), $R^2$ is preferably a C6-C14 aromatic hydrocarbon group or a bicyclic 9- to 10-membered fully unsaturated heterocyclic group having 1 to 3 heteroatoms selected from among a nitrogen atom, a sulfur atom, and an oxygen atoms, and $R^2$ may be substituted with $R^{21}$. When $R^2$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 4 to 8 carbon atoms (which may be substituted with a C1-C6 alkyl group) or a monocyclic 4- to 8-membered saturated or partially unsaturated heterocyclic ring having 1 to 3 heteroatoms selected from among a nitrogen atom, a sulfur atom, and an oxygen atom (which may be substituted with a C1-C6 alkyl group).

$R^{21}$ is a halogen atom, an aminocarbonyl group, a cyano group, a C1-C6 alkyl group (which may be substituted with a halogen atom), a C3-C6 cycloalkyl group, a C2-C6 alkynyl group (which may be substituted with a C6-C14 aromatic hydrocarbon group), a C6-C14 aromatic hydrocarbon group (which may be substituted with a group selected from the group consisting of a halogen atom and a C1-C6 alkoxy group), or a monocyclic or bicyclic 5- to 10-membered unsaturated heterocyclic ring having 1 to 3 heteroatoms selected from among a nitrogen atom, a sulfur atom, and an oxygen atom (which may be substituted with a group selected from the group consisting of a halogen atom, a C1-C6 alkyl group, a halogeno C1-C6 alkyl group, a C3-C6 cycloalkyl group, a C1-C6 alkoxy group, a C6-C14 aromatic hydrocarbon group, a nitrogen-containing saturated heterocyclic group, and a nitrogen-containing saturated heterocyclic carbonyl group).

In the compounds represented by Formula (I), $R^2$ is more preferably a C6-C14 aromatic hydrocarbon group, and $R^2$ may be substituted with $R^{21}$. When $R^2$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 4 to 8 carbon atoms (which may be substituted with a C1-C6 alkyl group).

$R^{21}$ is a halogen atom, a cyano group, a C1-C6 alkyl group (which may be substituted with a halogen atom), a C3-C6 cycloalkyl group, a phenyl group (which may be substituted with a group selected from the group consisting of a halogen atom a C1-C6 alkoxy group), or a monocyclic or bicyclic 5- to 10-membered unsaturated heterocyclic group having 1 to 3 heteroatoms selected from among a nitrogen atom, a sulfur atom, and an oxygen atom (which may be substituted with a group selected from the group consisting of a halogen atom, a C1-C6 alkyl group, a halogeno C1-C6 alkyl group, a C3-C6 cycloalkyl group, a C1-C6 alkoxy group, a morpholino group, a piperidinyl group, and a morpholinocarbonyl group).

In the compounds represented by Formula (I), also, $R^2$ is more preferably a C6-C10 aromatic hydrocarbon group, and $R^2$ may be substituted with $R^{21}$. When $R^2$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 5 or 6 carbon atoms (which may be substituted with a C1-C6 alkyl group); and

$R^{21}$ is a halogen atom, a C1-C6 alkyl group, or a monocyclic 5- or 6-membered unsaturated heterocyclic group having 1 to 3 nitrogen atoms (which may be substituted with a C1-C6 alkyl group).

In the compounds represented by Formula (I), also, $R^2$ is particularly preferably a phenyl group or a naphthyl group (which may be substituted with a group selected from the group consisting of a halogen atom and a C1-C6 alkyl group), an indanyl group (a 2,3-dihydro-1H-indenyl group), or a tetrahydronaphthyl group.

In the compounds represented by Formula (I), $R^3$ is a C6-C14 aromatic hydrocarbon group or a 5- to 10-membered fully unsaturated heterocyclic group.

The "C6-C14 aromatic hydrocarbon group" represented by $R^3$ is preferably a C6-C10 aromatic hydrocarbon group, more preferably a phenyl group or a naphthyl group, and particularly preferably a phenyl group.

The "5- to 10-membered fully unsaturated heterocyclic group" represented by $R^3$ is preferably a monocyclic or bicyclic 5- to 10-membered fully unsaturated heterocyclic group having 1 to 3 heteroatoms selected from among a

nitrogen atom, a sulfur atom, and an oxygen atom, more preferably a monocyclic or bicyclic 5- to 7-membered fully unsaturated heterocyclic group having 1 to 3 heteroatoms selected from among a nitrogen atom, a sulfur atom, and an oxygen atom, and particularly preferably a monocyclic 5- to 6-membered fully unsaturated heterocyclic group having 1 to 3 heteroatoms selected from among a nitrogen atom, a sulfur atom, and an oxygen atom. Such group is preferably an imidazolyl group, a pyridyl group, a thiophenyl group, an indolyl group, an indazolyl group, a benzopyranyl group, a benzotriazolyl group, a benzothiadiazolyl group, an isoxazolyl group, or a quinolyl group, more preferably an imidazolyl group, a pyridyl group, a thiophenyl group, an indolyl group, an indazolyl group, a benzopyranyl group, a benzotriazolyl group, a benzothiadiazolyl group, or a quinolyl group, more preferably a pyridyl group, a thiophenyl group, an indolyl group, an indazolyl group, a benzopyranyl group, a benzotriazolyl group, or a quinolyl group, and more preferably a pyridyl group.

In the compounds represented by Formula (I), $R^3$ may be unsubstituted or may be substituted. When $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be further substituted.

When $R^3$ is substituted, the position of substitution is not particularly limited. While the number of substituents is not particularly limited, it is preferably 0, i.e., unsubstituted. Alternatively, the number of substituents is preferably 1 to 4, and more preferably 1 to 3. When the number of substituent is 2 or more, the types of substituents may be the same with or different from each other.

In the compounds represented by Formula (I), $R^3$ may be preferably substituted with the above "substituent," and $R^3$ may be more preferably substituted with $R^{31}$. When $R^3$ has 2 substituents on the carbon atoms adjacent to each other on the aromatic hydrocarbon ring, the substituents may be preferably fused together with the carbon atoms to which they bind to form a 4- to 8-membered saturated or partially unsaturated hydrocarbon ring or heterocyclic ring, which may be substituted with Rc.

$R^{31}$, which can bind to $R^3$ as a substituent, is a halogen atom, a cyano group, a nitro group, a carboxyl group, a thioamide group, a C1-C6 alkyl group which may be substituted with Ra, an amino group which may be substituted with Ra, a C3-C6 cycloalkyl group which may be substituted with Rb, a C1-C6 alkoxy group which may be substituted with Rb, a C2-C7 alkoxycarbonyl group, a C1-C14 acyl group which may be substituted with Rb, a C6-C14 aromatic hydrocarbon group which may be substituted with Rb, a 5- to 10-membered unsaturated heterocyclic group which may be substituted with Rc, an aminocarbonyl group which may be substituted with Rd and Re, or $-S(=O)_2Rf$.

While the number of substituent $R^{31}$ is not particularly limited, it is preferably 0, i.e., unsubstituted. Alternatively, the number of substituents is preferably 1 to 4, and more preferably 1 to 3. When the number of substituent $R^{31}$is 2 or more, the types of substituents may be the same with or different from each other.

The "halogen atom" represented by $R^{31}$ is preferably a fluorine atom, a chlorine atom, or a bromine atom, and more preferably a chlorine atom or a bromine atom.

The "C1-C6 alkyl group" in the "C1-C6 alkyl group which may be substituted with Ra" represented by $R^{31}$ is preferably a C1-C3 alkyl group, and more preferably a methyl group.

The substituent Ra in the "C1-C6 alkyl group which may be substituted with Ra" represented by $R^{31}$ is a halogen atom, a hydroxy group, a C1-C14 acyl group, a C1-C14 acyloxy group, a C2-C6 alkynyl group, or a C1-C6 alkoxy C1-C6 alkoxy group.

The "halogen atom" represented by Ra is preferably a fluorine atom.

The "C1-C14 acyl group" represented by Ra is preferably an acetyl group.

The "C1-C14 acyloxy group" represented by Ra is preferably an acetyloxy group.

The "C2-C6 alkynyl group" represented by Ra is preferably an ethynyl group or a 1-propynylgroup.

The "C1-C6 alkoxy C1-C6 alkoxy group" represented by Ra is preferably a methoxymethoxy group.

The substituent Ra in the "C1-C6 alkyl group which may be substituted with Ra" represented by $R^{31}$ is preferably a halogen atom, a hydroxy group, a C1-C6 acyloxy group, a C2-C6 alkynyl group, or a C1-C6 alkoxy C1-C6 alkoxy group, and more preferably a halogen atom or a hydroxy group. While the number of Ra to bind to a C1-C6 alkyl group as a substituent is not particularly limited, it is preferably 0, i.e., unsubstituted, or 1 or more. When the number of the substituent Ra is 2 or more, the types of substituents may be the same with or different from each other.

The substituent Ra in the "amino group which may be substituted with Ra" represented by $R^{31}$ is, as described above, a halogen atom, a hydroxy group, a C1-C14 acyl group, a C1-C 14 acyloxy group, a C2-C6 alkynyl group, or a C1-C6 alkoxy C1-C6 alkoxy group, preferably a C1-C14 acyl group, and more preferably an acetyl group. While the number of Ra to bind to an amino group as a substituent is not particularly limited, it is preferably 0, i.e., unsubstituted, or it is preferably 1, and more preferably 0.

The "C3-C6 cycloalkyl group" in the "C3-C6 cycloalkyl group which may be substituted with Rb" represented by $R^{31}$ is preferably a cyclopropyl group.

The substituent Rb in the "C3-C6 cycloalkyl group which may be substituted with Rb" represented by $R^{31}$ is a halogen atom, an amino group, or a C1-C6 alkoxy group.

The "halogen atom" represented by Rb is preferably a fluorine atom.

The "C1-C6 alkoxy group" represented by Rb is preferably a C1-C3 alkoxy group, and more preferably a methoxy group.

The substituent Rb in the "C3-C6 cycloalkyl group which may be substituted with Rb" represented by $R^{31}$ is preferably an amino group. While the number of Rb to bind to a C3-C6 cycloalkyl group as a substituent is not particularly limited, it is preferably 0, i.e., unsubstituted, or preferably 1. When the number of substituent Rb is 2 or more, the types of substituents may be the same with or different from each other.

The "C1-C6 alkoxy group" in the "C1-C6 alkoxy group which may be substituted with Rb" represented by $R^{31}$ is preferably a C1-C3 alkoxy group, and more preferably a methoxy group.

The substituent Rb in the "C1-C6 alkoxy group which may be substituted with Rb" represented by $R^{31}$ is, as described above, a halogen atom, an amino group, or a C1-C6 alkoxy group, preferably a halogen atom, and more preferably a fluorine atom. While the number of Rb to bind to a C1-C6 alkoxy group as a substituent is not particularly limited, it is 0, i.e., unsubstituted, or 1 or 2. When the number of substituent Rb is 2 or more, the types of substituents may be the same with or different from each other.

The "C2-C7 alkoxycarbonyl group" represented by $R^{31}$ is preferably a C2-C4 alkoxycarbonyl group, and more preferably a methoxycarbonyl group.

The "C1-C14 acyl group" in the "C1-C14 acyl group which may be substituted with Rb" represented by $R^{31}$ is preferably an acetyl group.

The substituent Rb in the "C1-C14 acyl group which may be substituted with Rb" represented by $R^{31}$ is, as described above, a halogen atom, an amino group, or a C1-C6 alkoxy group, preferably a halogen atom, and more preferably a fluorine atom. While the number of Rb to bind to a C1-C14 acyl group as a substituent is not particularly limited, it is 0, i.e., unsubstituted, or it is 1 to 3. When the number of substituent Rb is 2 or more, the types of substituents may be the same with or different from each other.

The "thioamide group" represented by $R^{31}$ is preferably $-C(=S)-NH_2$.

The "C6-C14 aromatic hydrocarbon group" in the "C6-C14 aromatic hydrocarbon group which may be substituted with Rb" represented by $R^{31}$ is preferably a C6-C10 aromatic hydrocarbon group, and more preferably a phenyl group.

The substituent Rb in the "C6-C14 aromatic hydrocarbon group which may be substituted with Rb" represented by $R^{31}$ is, as described above, a halogen atom, an amino group, or a C1-C6 alkoxy group, preferably a halogen atom or a C1-C3 alkoxy group, more preferably a halogen atom, and more preferably a fluorine atom. While the number of Rb to bind to a C6-C14 aromatic hydrocarbon group as a substituent is not particularly limited, it is preferably 0, i.e., unsubstituted, or it is 1. When the number of substituent Rb is 2 or more, the types of substituents may be the same with or different from each other.

The "5- to 10-membered unsaturated heterocyclic group" in the "5- to 10-membered unsaturated heterocyclic group which may be substituted with Rc" represented by $R^{31}$ is preferably a monocyclic or bicyclic 5- to 10-membered fully or partially unsaturated heterocyclic group having 1 to 4 heteroatoms selected from among a nitrogen atom, a sulfur atom, and an oxygen atom, and more preferably a monocyclic 5- to 6-membered unsaturated heterocyclic group having 1 to 4 heteroatoms selected from among a nitrogen atom, a sulfur atom, and an oxygen atom. Such group is preferably a pyrrolyl group, an imidazolyl group, a pyrazolyl group, a tetrazolyl group, an isoxazolyl group, an oxadiazolyl group, or a dihydroxadiazolyl group, and more preferably a pyrazolyl group, a 1,3,4-oxadiazolyl group, or a 2,3-dihydro-1,3,4-oxazolyl group.

The substituent Rc in the "5- to 10-membered unsaturated heterocyclic group, which may be substituted with 1 or more substituents Rc" represented by $R^{31}$, is a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, a C1-C14 acyloxy group, or a C7-C13 aralkyloxy group.

The "halogen atom" represented by Rc is preferably a fluorine atom.

The "C1-C6 alkyl group which may be substituted with a hydroxy group" represented by Rc is preferably a C1-C3 alkyl group which may be substituted with a hydroxy group, and more preferably a methyl group or a hydroxyethyl group.

The "halogeno C1-C6 alkyl group" represented by Rc is preferably a halogeno C1-C3 alkyl group, and more preferably a trifluoromethyl group or a difluoroethyl group.

The "C1-C14 acyl group" represented by Rc is preferably an acetyl group or a cyclopropanoyl group.

The "C1-C14 acylamino group" represented by Rc is preferably an acetylamino group.

The "C1-C14 acyloxy group" represented by Rc is preferably an acetyloxy group.

The "C7-C13 aralkyloxy group" represented by Rc is preferably a benzyloxy group.

The substituent Rc in the "5- to 10-membered unsaturated heterocyclic group which may be substituted with Rc" represented by $R^{31}$, is preferably a halogen atom, a C1-C6 alkyl group, or an oxo group, more preferably a C1-C6 alkyl group or an oxo group, and more preferably a C1-C6 alkyl group. While the number of Rc to bind to a 5- to 10-membered unsaturated heterocyclic group as a substituent is not particularly limited, it is preferably 0, i.e., unsubstituted,

or it is preferably 1 or more, and more preferably 0. When the number of the substituent Rc is 2 or more, the types of substituents may be the same with or different from each other.

The "aminocarbonyl group which may be substituted with Rd and Re" represented by $R^{31}$ is specifically represented by Formula (II) below.

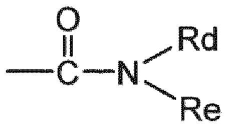

(II)

Rd and Re are the same or different and represent a hydrogen atom, a hydroxy group, a C7-C13 aralkyloxy group, or a C1-C6 alkyl group which may be substituted with a hydroxyl group. Alternatively, Rd and Re form, together with the nitrogen atoms adjacent thereto, a 4-to 10-membered saturated or unsaturated heterocyclic group which may be substituted with an amino group, a spiroheterocyclic group, or a bridged heterocyclic group.

The "C7-C13 aralkyloxy group" represented by Rd or Re is preferably a benzyloxy group.

The "C1-C6 alkyl group which may be substituted with a hydroxy group" represented by Rd or Re is preferably a C1-C3 alkyl group which may be substituted with a hydroxy group, and more preferably a methyl group or a hydroxy ethyl group.

The "saturated heterocyclic group" in the "4- to 10-membered saturated heterocyclic group which may be substituted with an amino group" formed by Rd or Re together with a nitrogen atom adjacent thereto is preferably a monocyclic or bicyclic 4- to 10-membered saturated heterocyclic group having 1 to 3 heteroatoms selected from among a nitrogen atom, a sulfur atom, and an oxygen atom, more preferably a monocyclic 5- to 6-membered saturated heterocyclic group having 1 to 3 heteroatoms selected from among a nitrogen atom, a sulfur atom, and an oxygen atom, and particularly preferably an azetidinyl group, a pyrrolidinyl group, a piperidino group, a piperazinyl group, or a morpholino group.

The "unsaturated heterocyclic group" in the "4- to 10-membered saturated or unsaturated heterocyclic group which may be substituted with an amino group" formed by Rd or Re together with a nitrogen atom adjacent thereto is preferably a monocyclic or bicyclic 5-to 10-membered unsaturated heterocyclic group having 1 to 3 heteroatoms selected from among a nitrogen atom, a sulfur atom, and an oxygen atom, more preferably a monocyclic 5- to 6-membered unsaturated heterocyclic group having 1 to 3 heteroatoms selected from among a nitrogen atom, a sulfur atom, and an oxygen atom, and particularly preferably a pyrrolyl group.

The "spiroheterocyclic group" formed by Rd or Re together with a nitrogen atom adjacent thereto is preferably a monospiroheterocyclic group, and more preferably an oxoazaspirononanylcarbamoyl group or an azaspirooctanylcarbamoyl group.

The "bridged heterocyclic group" formed by Rd or Re together with a nitrogen atom adjacent thereto is preferably a bicyclic bridged heterocyclic group, and more preferably an oxoazabicyclooctanylcarbamoyl group.

The substituents Rd and Re in the "aminocarbonyl group which may be substituted with Rd and Re" represented by $R^{31}$ are preferably the same or different, represent a hydroxy group or a C1-C6 alkyl group, or Rd and Re form, together with the nitrogen atoms adjacent thereto, a monocyclic 5- to 6-membered saturated heterocyclic group, a monospiroheterocyclic group, or a bicyclic bridged heterocyclic group, having 1 to 3 heteroatoms selected from among a nitrogen atom, a sulfur atom, and an oxygen atom, which may be substituted with an amino group.

The "aminocarbonyl group which may be substituted with Rd and Re" represented by $R^{31}$ is preferably a $-CONH_2$ group, a (mono- or di-C1-C6 alkyl)aminocarbonyl group, a hydroxyaminocarbonyl group, a (C7-C13 aralkyl)oxyaminocarbonyl group, or a cyclic aminocarbonyl group, more preferably a $-CONH_2$ group, a (mono- or di-Cl-C3 alkyl)aminocarbonyl group, a hydroxyaminocarbonyl group, a benzyloxyaminocarbonyl group, a pyrrolidin-1-ylcarbonyl group, a piperidin-1-ylcarbonyl group, a piperazin-1-ylcarbonyl group, a morpholin-4-ylcarbonyl group, an azetidin-1-ylcarbonyl group, an oxoazabicyclooctanylcarbonyl group, an oxoazaspirononanylcarbonyl group, or an azaspirooctanylcarbonyl group, and particularly preferably a $-CONH_2$ group, a dimethylaminocarbonyl group, or a pyrrolidin-1-ylcarbonyl group.

Rf in the "$-S(=O)_2Rf$" represented by $R^{31}$ is an amino group, a C1-C6 alkyl group, or a 4- to 10-membered saturated heterocyclic group.

The "C1-C6 alkyl group" represented by Rf is preferably a C1-C3 alkyl group, and more preferably a methyl group.

The "4- to 10-membered saturated heterocyclic group" represented by Rf is preferably a monocyclic or bicyclic 4- to 10-membered saturated heterocyclic group having 1 to 3 heteroatoms selected from among a nitrogen atom, a sulfur atom, and an oxygen atom, more preferably a monocyclic 5- to 6-membered saturated heterocyclic group having 1 to 3 heteroatoms selected from among a nitrogen atom, a sulfur atom, and an oxygen atom, and particularly preferably a pyrrolidinyl group, a piperidino group, or a piperazinyl group.

The "$-S(=O)_2Rf$" represented by $R^{31}$ is preferably an aminosulfonyl group, a methylsulfonyl group, or a pipe-

ridinosulfonyl group.

$R^{31}$, which may bind to $R^3$ as a substituent, is preferably a halogen atom, a cyano group, a nitro group, a carboxyl group, a thioamide group, a C1-C6 alkyl group (which may be substituted with a group selected from the group consisting of a halogen atom, a hydroxy group, a C1-C14 acyl group, a C1-C14 acyloxy group, a C2-C6 alkynyl group, and a C1-C6 alkoxy C1-C6 alkoxy group), an amino group (which may be substituted with a C1-C14 acyl group), a C3-C6 cycloalkyl group (which may be substituted with an amino group), a C1-C6 alkoxy group (which may be substituted with a halogen atom), a C2-C7 alkoxycarbonyl group, a C1-C14 acyl group (which may be substituted with a halogen atom), a C6-C14 aromatic hydrocarbon group (which may be substituted with a group selected from the group consisting of a halogen atom, an amino group, and a C1-C6 alkoxy group), a monocyclic or bicyclic 5- to 10-membered unsaturated heterocyclic group having 1 to 4 heteroatoms selected from among a nitrogen atom, a sulfur atom, and an oxygen atom (which may be substituted with a group selected from the group consisting of a halogen atom, an oxo group, and a C1-C6 alkyl group), an aminocarbonyl group which may be substituted with Rd and Re (wherein Rd and Re are the same or different, and represent a hydrogen atom, a hydroxy group, a C7-C13 aralkyloxy group, or a C1-C6 alkyl group which may be substituted with a hydroxyl group, or Rd and Re form, together with the nitrogen atoms adjacent thereto, a monocyclic or bicyclic 4- to 10-membered saturated or unsaturated heterocyclic group, a spiroheterocyclic group, or a bridged heterocyclic group, having 1 to 3 heteroatoms selected from among a nitrogen atom, a sulfur atom, and an oxygen atom, which may be substituted with an amino group), or $-S(=O)_2Rf$ (wherein Rf is an amino group, a C1-C6 alkyl group, or a 4- to 10-membered saturated heterocyclic group).

$R^{31}$ is more preferably a halogen atom, a cyano group, a nitro group, a carboxyl group, a thioamide group, a C1-C6 alkyl group (which may be substituted with a group selected from the group consisting of a halogen atom, a hydroxy group, a C1-C14 acyloxy group, a C2-C6 alkynyl group, and a C1-C6 alkoxy C1-C6 alkoxy group), an amino group, a C3-C6 cycloalkyl group (which may be substituted with an amino group), a C1-C6 alkoxy group (which may be substituted with a halogen atom), a C2-C7 alkoxycarbonyl group, a C1-C14 acyl group (which may be substituted with a halogen atom), a C6-C10 aromatic hydrocarbon group (which may be substituted with a halogen atom), a monocyclic or bicyclic 5- to 10-membered unsaturated heterocyclic group having 1 to 4 heteroatoms selected from among a nitrogen atom, a sulfur atom, and an oxygen atom (which may be substituted with a group selected from the group consisting of a C1-C6 alkyl group and an oxo group), a $-CONH_2$ group, a (mono- or di-C1-C6 alkyl)aminocarbonyl group, a hydroxyaminocarbonyl group, a (C7-C13 aralkyl)oxyaminocarbonyl group, a cyclic aminocarbonyl group, an aminosulfonyl group, a C1-C6 alkylsulfonyl group, or a piperidinosulfonyl group.

$R^{31}$ is more preferably a halogen atom, an amino group, a C1-C6 alkyl group (which may be substituted with a group selected from the group consisting of a halogen atom and a hydroxy group), a C1-C6 alkoxy group (which may be substituted with a halogen atom), a monocyclic 5- or 6-membered unsaturated heterocyclic group having 1 to 4 heteroatoms selected from among a nitrogen atom, a sulfur atom, and an oxygen atom, a $-CONH_2$ group, a (mono- or di-C1-C6 alkyl)aminocarbonyl group, or a hydroxyaminocarbonyl group.

$R^{31}$ is more preferably a halogen atom, an amino group, a C1-C6 alkoxy group, or a $-CONH_2$ group.

In the compounds represented by Formula (I), when there are 2 or more substituents for $R^3$ and 2 substituents are on the carbon atoms adjacent to each other on the aromatic hydrocarbon ring of $R^3$, the "4- to 8-membered saturated or partially unsaturated hydrocarbon ring or heterocyclic ring which may be substituted" formed by the substituents together with the carbon atoms to which they bind is a ring that is fused to the aromatic hydrocarbon ring, for example, a benzene ring. The "4- to 8-membered saturated or partially unsaturated hydrocarbon ring or heterocyclic ring" in the "4- to 8-membered saturated or partially unsaturated hydrocarbon ring or heterocyclic ring which may be substituted" is preferably a monocyclic saturated or partially unsaturated hydrocarbon ring having 4 to 8 carbon atoms, or a monocyclic 4- to 8-membered saturated or partially unsaturated heterocyclic ring having 1 to 4 heteroatoms selected from among a nitrogen atom, a sulfur atom, and an oxygen atom, more preferably a monocyclic 4- to 6-membered saturated or partially unsaturated heterocyclic ring having 1 to 3 heteroatoms selected from among a nitrogen atom, a sulfur atom, and an oxygen atom, and further preferably a monocyclic 6-membered saturated or partially unsaturated heterocyclic ring having 1 or 2 oxygen atoms.

The substituent Rc in the "4- to 8-membered saturated or partially unsaturated hydrocarbon ring or heterocyclic ring which may be substituted with Rc" is, as described above, a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, a C1-C14 acyloxy group, or a C7-C13 aralkyloxy group, preferably a hydroxy group, an amino group, an oxo group, a C1-C6 alkyl group which may be substituted with a hydroxy group, a halogeno C1-C6 alkyl group, a C1-C14 acyl group, or a C1-C14 acyloxy group, and more preferably a hydroxy group or a C1-C6 alkyl group. While the number of Rc which may bind to a saturated or partially unsaturated hydrocarbon ring or heterocyclic ring as a substituent is not particularly limited, it is preferably 1 to 3. When the number of substituent Rc is 2 or more, the types of substituents may be the same with or different from each other.

The "4- to 8-membered saturated or partially unsaturated hydrocarbon ring or heterocyclic ring which may be substituted with Rc" is preferably a monocyclic saturated or partially unsaturated hydrocarbon ring having 4 to 8 carbon

atoms (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, a C1-C14 acyloxy group, and a C7-C13 aralkyloxy group) or a monocyclic 4- to 8-membered saturated or partially unsaturated heterocyclic ring having 1 to 4 heteroatoms selected from among a nitrogen atom, a sulfur atom, and an oxygen atom (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, a C1-C14 acyloxy group, and a C7-C13 aralkyloxy group).

Such a ring is more preferably a monocyclic saturated or partially unsaturated hydrocarbon ring having 4 to 8 carbon atoms (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, and a C1-C14 acyloxy group) or a monocyclic 4- to 8-membered saturated or partially unsaturated heterocyclic ring having 1 to 3 heteroatoms selected from among a nitrogen atom, a sulfur atom, and an oxygen atom (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, and a C1-C14 acyloxy group).

Such a ring is more preferably a monocyclic 4- to 6-membered saturated or partially unsaturated heterocyclic ring having 1 to 3 heteroatoms selected from among a nitrogen atom, a sulfur atom, and an oxygen atom (which may be substituted with a group selected from the group consisting of a hydroxy group, an amino group, an oxo group, a C1-C6 alkyl group, a halogeno C1-C6 alkyl group, a C1-C14 acylamino group, and a C1-C14 acyloxy group).

Such a ring is more preferably a monocyclic 6-membered saturated or partially unsaturated heterocyclic ring having 1 or 2 oxygen atoms (which may be substituted with a group selected from the group consisting of a hydroxy group and a C1-C6 alkyl group).

In the compounds represented by Formula (I), the fused ring formed when there are 2 substituents on the carbon atoms adjacent to each other on the aromatic hydrocarbon ring of $R^3$ is, for example, a chroman ring, a dihydrobenzoxazine ring, a dihydroindene ring, an indoline ring, a tetrahydroquinoxaline ring, a dihydrobenzodioxane ring, a tetrahydronaphthalene ring, a tetrahydroquinoline ring, a tetrahydroisoquinoline ring, a dihydrobenzothiophene ring, an isoindoline ring, a dihydroisobenzofuran ring, a dihydrobenzoimidazole ring, or the like.

In the compounds represented by Formula (I), $R^3$ is preferably a C6-C14 aromatic hydrocarbon group or a monocyclic or bicyclic 5- to 10-membered fully unsaturated heterocyclic group having 1 to 3 heteroatoms selected from among a nitrogen atom, a sulfur atom, and an oxygen atom. $R^3$ may be substituted with $R^{31}$. When $R^3$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 4 to 8 carbon atoms (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, a C1-C14 acyloxy group, and a C7-C13 aralkyloxy group) or a monocyclic 4- to 8-membered saturated or partially unsaturated heterocyclic ring having 1 to 4 heteroatoms selected from among a nitrogen atom, a sulfur atom, and an oxygen atom (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, a C1-C14 acyloxy group, and a C7-C13 aralkyloxy group).

$R^{31}$ is a halogen atom, a cyano group, a nitro group, a carboxyl group, a thioamide group, a C1-C6 alkyl group (which may be substituted with a group selected from the group consisting of a halogen atom, a hydroxy group, a C1-C14 acyl group, a C1-C14 acyloxy group, a C2-C6 alkynyl group, and a C1-C6 alkoxy C1-C6 alkoxy group), an amino group (which may be substituted with a C1-C14 acyl group), a C3-C6 cycloalkyl group (which may be substituted with an amino group), a C1-C6 alkoxy group (which may be substituted with a halogen atom), a C2-C7 alkoxycarbonyl group, a C1-C14 acyl group (which may be substituted with a halogen atom), a C6-C14 aromatic hydrocarbon group (which may be substituted with a group selected from the group consisting of a halogen atom, an amino group, and a C1-C6 alkoxy group), a monocyclic or bicyclic 5- to 10-membered unsaturated heterocyclic group having 1 to 4 heteroatoms selected from among a nitrogen atom, a sulfur atom, and an oxygen atom (which may be substituted with a group selected from the group consisting of a halogen atom, an oxo group, and a C1-C6 alkyl group), an aminocarbonyl group which may be substituted with Rd and Re (wherein Rd and Re are the same or different, represent a hydrogen atom, a hydroxy group, a C7-C13 aralkyloxy group, or a C1-C6 alkyl group which may be substituted with a hydroxyl group, or Rd and Re form, together with the nitrogen atoms adjacent thereto, a monocyclic or bicyclic 4- to 10-membered saturated or unsaturated heterocyclic group, a spiroheterocyclic group, or a bridged heterocyclic group, having 1 to 3 heteroatoms selected from among a nitrogen atom, a sulfur atom, and an oxygen atom, which may be substituted with an amino group), or $-S(=O)_2Rf$ (wherein Rf is an amino group, a C1-C6 alkyl group, or a 4- to 10-membered saturated heterocyclic

group).

In the compounds represented by Formula (I), $R^3$ is more preferably a C6-C10 aromatic hydrocarbon group or a monocyclic or bicyclic 5- to 10-membered fully unsaturated heterocyclic group having 1 to 3 heteroatoms selected from among a nitrogen atom, a sulfur atom, and an oxygen atom. $R^3$ may be substituted with $R^{31}$, and when it has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 4 to 8 carbon atoms (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, and a C1-C14 acyloxy group) or a monocyclic 4- to 8-membered saturated or partially unsaturated heterocyclic ring having 1 to 3 heteroatoms selected from among a nitrogen atom, a sulfur atom, and an oxygen atom (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1 -C14 acyl group, a C1-C14 acylamino group, and a C1-C14 acyloxy group).

$R^{31}$ is a halogen atom, a cyano group, a nitro group, a carboxyl group, a thioamide group, a C1-C6 alkyl group (which may be substituted with a group selected from the group consisting of a halogen atom, a hydroxy group, a C1-C14 acyloxy group, a C2-C6 alkynyl group, and a C1-C6 alkoxy C1-C6 alkoxy group), an amino group, a C3-C6 cycloalkyl group (which may be substituted with an amino group), a C1-C6 alkoxy group (which may be substituted with a halogen atom), a C2-C7 alkoxycarbonyl group, a C1-C14 acyl group (which may be substituted with a halogen atom), a C6-C10 aromatic hydrocarbon group (which may be substituted with a halogen atom), a monocyclic or bicyclic 5- to 10-membered unsaturated heterocyclic group having 1 to 4 heteroatoms selected from among a nitrogen atom, a sulfur atom, and an oxygen atom (which may be substituted with a group selected from the group consisting of a C1-C6 alkyl group and an oxo group), a $-CONH_2$ group, a (mono- or di-C1-C6 alkyl)aminocarbonyl group, a hydroxyaminocarbonyl group, a (C7-C13 aralkyl)oxyaminocarbonyl group, a cyclic aminocarbonyl group, an aminosulfonyl group, a C1-C6 alkylsulfonyl group, or a piperidinosulfonyl group.

In the compounds represented by Formula (I), $R^3$ is more preferably a C6-C10 aromatic hydrocarbon group (wherein the C6-C10 aromatic hydrocarbon group may be substituted with $R^{31}$ or, when it has 2 substituents on the carbon atoms adjacent to each other on the aromatic hydrocarbon ring, it may be fused together with the carbon atoms to which they bind to form a monocyclic 4- to 6-membered saturated or partially unsaturated heterocyclic ring having 1 to 3 heteroatoms selected from among a nitrogen atom, a sulfur atom, and an oxygen atom (which may be substituted with a group selected from the group consisting of a hydroxy group, an amino group, an oxo group, a C1-C6 alkyl group, a halogeno C1-C6 alkyl group, a C1-C14 acylamino group, and a C1-C14 acyloxy group) or a monocyclic 5- to 6-membered fully unsaturated heterocyclic group having 1 to 3 heteroatoms selected from among a nitrogen atom, a sulfur atom, and an oxygen atom (which may be substituted with a group selected from the group consisting of a halogen atom, a C1-C6 alkyl group which may be substituted with a hydroxyl group, a C1-C6 alkoxy group, a C2-C7 alkoxycarbonyl group, a $-CONH_2$ group, a (mono- or di-C1-C6 alkyl)aminocarbonyl group, a pyrrolidin-1-ylcarbonyl group, a morpholin-4-ylcarbonyl group, a 2-oxa-7-azaspiro[3.5]nonanyl group, a 3-oxa-8-azabicyclo[3.2.1]octanyl group, and an 8-oxa-3-azabicyclo[3.2.1]octanyl group).

$R^{31}$ is a halogen atom, an amino group, a C1-C6 alkyl group (which may be substituted with a group selected from the group consisting of a halogen atom and a hydroxy group), a C1-C6 alkoxy group (which may be substituted with a halogen atom), a monocyclic 5- or 6-membered unsaturated heterocyclic group having 1 to 4 heteroatoms selected from among a nitrogen atom, a sulfur atom, and an oxygen atom, a $-CONH_2$ group, a (mono- or di-C1-C6 alkyl)aminocarbonyl group, or a hydroxyaminocarbonyl group.

In the compounds represented by Formula (I), also, $R^3$ is particularly preferably a phenyl group (which may be substituted with $R^{31}$ or, when the phenyl group has 2 substituents on the carbon atoms adjacent to each other on the benzene ring, the substituents may be fused together with the carbon atoms to which they bind to form a monocyclic 6-membered saturated or partially unsaturated heterocyclic ring having 1 or 2 oxygen atoms (which may be substituted with a group selected from the group consisting of a hydroxy group and a C1-C6 alkyl group)) or a pyridyl group (which may be substituted with a $-CONH_2$ group, a (mono or di C1-C6 alkyl)aminocarbonyl group, or a pyrrolidin-1-yl carbonyl group).

$R^{31}$ is a halogen atom, an amino group, a C1-C6 alkoxy group, or a $-CONH_2$ group.

In the compounds represented by Formula (I), $R^4$ is a hydrogen atom or a C1-C6 alkyl group.

The "C1-C6 alkyl group" represented by $R^4$ is preferably a C1-C3 alkyl group, and more preferably a methyl group.

$R^4$ is preferably a hydrogen atom or a methyl group, and more preferably a hydrogen atom.

Preferable examples of the sulfonamide compounds represented by Formula (I) or salts thereof include those as described below:

In Formula (I),

$X^1$ represents an oxygen atom or a sulfur atom;
$X^2$ represents an oxygen atom;
$X^3$ represents -NH-;
$X^4$ represents a hydrogen atom or a methyl group;
$R^1$ represents $-C(R^{11})(R^{12})-$ (wherein $R^{11}$ and $R^{12}$ are the same or different and represent a hydrogen atom or a C1-C6 alkyl group);
$R^2$ represents a C6-C14 aromatic hydrocarbon group, and may be substituted with $R^{21}$ and, when $R^2$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 4 to 8 carbon atoms (which may be substituted with a C1-C6 alkyl group);
$R^{21}$ is a halogen atom, a cyano group, a C1-C6 alkyl group (which may be substituted with a halogen atom), a C3-C6 cycloalkyl group, a phenyl group (which may be substituted with a group selected from the group consisting of a halogen atom and a C1-C6 alkoxy group), or a monocyclic or bicyclic 5- to 10-membered unsaturated heterocyclic group having 1 to 3 heteroatoms selected from among a nitrogen atom, a sulfur atom, and an oxygen atom (which may be substituted with a group selected from the group consisting of a halogen atom, a C1-C6 alkyl group, a halogeno C1-C6 alkyl group, a C3-C6 cycloalkyl group, a C1-C6 alkoxy group, a morpholino group, a piperidinyl group, and a morpholinocarbonyl group);
$R^3$ is a C6-C10 aromatic hydrocarbon group or a monocyclic or bicyclic 5- to 10-membered fully unsaturated heterocyclic group having 1 to 3 heteroatoms selected from among a nitrogen atom, a sulfur atom, and an oxygen atom. $R^3$ may be substituted with $R^{31}$, and, when $R^3$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 4 to 8 carbon atoms (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1-C14 acyl group, a C1-C14 acylamino group, and a C1-C14 acyloxy group) or a monocyclic 4- to 8-membered saturated or partially unsaturated heterocyclic ring having 1 to 3 heteroatoms selected from among a nitrogen atom, a sulfur atom, and an oxygen atom (which may be substituted with a group selected from the group consisting of a halogen atom, a hydroxy group, an amino group, an oxo group, a C1-C6 alkyl group which may be substituted with a hydroxy group, a halogeno C1-C6 alkyl group, a C1 -C14 acyl group, a C1-C14 acylamino group, and a C1-C14 acyloxy group);
$R^{31}$ is a halogen atom, a cyano group, a nitro group, a carboxyl group, a thioamide group, a C1-C6 alkyl group (which may be substituted with a group selected from the group consisting of a halogen atom, a hydroxy group, a C1-C14 acyloxy group, a C2-C6 alkynyl group, and a C1-C6 alkoxy C1-C6 alkoxy group), an amino group, a C3-C6 cycloalkyl group (which may be substituted with an amino group), a C1-C6 alkoxy group (which may be substituted with a halogen atom), a C2-C7 alkoxycarbonyl group, a C1-C14 acyl group (which may be substituted with a halogen atom), a C6-C10 aromatic hydrocarbon ring (which may be substituted with a halogen atom), a monocyclic or bicyclic 5- to 10-membered unsaturated heterocyclic group having 1 to 4 heteroatoms selected from among a nitrogen atom, a sulfur atom, and an oxygen atom (which may be substituted with a group selected from the group consisting of a C1-C6 alkyl group and an oxo group), a $-CONH_2$ group, a (mono- or di-C1-C6 alkyl)aminocarbonyl group, a hydroxyaminocarbonyl group, a (C7-C13 aralkyloxy)oxyaminocarbonyl group, a cyclic aminocarbonyl group, an aminosulfonyl group, a C1-C6 alkylsulfonyl group, or a piperidinosulfonyl group; and
$R^4$ represents a hydrogen atom,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

More preferable examples of the sulfonamide compounds represented by Formula (I) or salts thereof include those as described below:
In Formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$ (wherein $R^{11}$ represents a C1-C6 alkyl group, and $R^{12}$ represents a hydrogen atom);
$R^2$ represents a C6-C10 aromatic hydrocarbon group, and may be substituted with $R^{21}$, and, when $R^2$ has 2 substituents on the carbon atoms adjacent to each other on the aromatic hydrocarbon ring, the substituents may be fused together with the carbon atoms to which they bind to form a monocyclic saturated or partially unsaturated

hydrocarbon ring having 5 or 6 carbon atoms (which may be substituted with a C1-C6 alkyl group);
$R^{21}$ is a halogen atom, a C1-C6 alkyl group, or a monocyclic 5- or 6-membered unsaturated heterocyclic group having 1 to 3 nitrogen atoms (which may be substituted with a C1-C6 alkyl group);
$R^3$ is a C6-C10 aromatic hydrocarbon group (wherein the C6-C10 aromatic hydrocarbon group may be substituted with $R^{31}$, and, when the C6-C10 aromatic hydrocarbon group has 2 substituents on the carbon atoms adjacent to each other on the aromatic hydrocarbon ring, the substituents may be fused together with the carbon atoms to which they bind to form a monocyclic 4- to 6-membered saturated or partially unsaturated heterocyclic ring having 1 to 3 heteroatoms selected from among a nitrogen atom, a sulfur atom, and an oxygen atom (which may be substituted with a group selected from the group consisting of a hydroxy group, an amino group, an oxo group, a C1-C6 alkyl group, a halogeno C1-C6 alkyl group, a C1-C14 acylamino group, and C1-C14 acyloxy group) or a monocyclic 5- to 6-membered fully unsaturated heterocyclic group having 1 to 3 heteroatoms selected from among a nitrogen atom, a sulfur atom, and an oxygen atom (which may be substituted with a group selected from the group consisting of a halogen atom, a C1-C6 alkyl group which may be substituted with a hydroxyl group, a C1-C6 alkoxy group, a C2-C7 alkoxycarbonyl group, a $-CONH_2$ group, a (mono- or di-C1-C6 alkyl)aminocarbonyl group, a pyrrolidin-1-ylcarbonyl group, a morpholin-4-ylcarbonyl group, a 2-oxa-7-azaspiro[3.5]nonanyl group, a 3-oxa-8-azabicyclo[3.2.1]octanyl group, and an 8-oxa-3-azabicyclo[3.2.1]octanyl group);
$R^{31}$ is a halogen atom, an amino group, a C1-C6 alkyl group (which may be substituted with a group selected from the group consisting of a halogen atom and a hydroxy group), a C1-C6 alkoxy group (which may be substituted with a halogen atom), a monocyclic 5- to 6-membered unsaturated heterocyclic group having 1 to 4 heteroatoms selected from among a nitrogen atom, a sulfur atom, and an oxygen atom, a $-CONH_2$ group, a (mono- or di-C1-C6 alkyl)aminocarbonyl group, or a hydroxyaminocarbonyl group; and
$R^4$ represents a hydrogen atom.

More preferable examples of the sulfonamide compounds represented by Formula (I) or salts thereof include those as described below:
In Formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$ (wherein $R^{11}$ represents a methyl group, and $R^{12}$ represents a hydrogen atom);
$R^2$ represents a phenyl group or a naphthyl group, and may be substituted with $R^{21}$, and, when $R^2$ has 2 substituents on the carbon atoms adjacent to each other on the aromatic hydrocarbon ring, the substituents may be fused together with the carbon atoms to which they bind to form a monocyclic saturated or partially unsaturated hydrocarbon ring having 5 or 6 carbon atoms (which may be substituted with a C1-C6 alkyl group);
$R^{21}$ is a halogen atom or a C1-C6 alkyl group;
$R^3$ is a phenyl group (wherein the phenyl group may be substituted with $R^{31}$, and when the phenyl group has 2 substituents on the carbon atoms adjacent to each other on the benzene ring, the substituents may be fused together with the carbon atoms to which they bind to form a monocyclic 6-membered saturated or partially unsaturated heterocyclic ring having 1 or 2 oxygen atoms (which may be substituted with a group selected from the group consisting of a hydroxyl group and a C1-C6 alkyl group), or a pyridyl group (which may be substituted with a $-CONH_2$ group, a (mono- or di-C1-C6 alkyl)aminocarbonyl group, or a pyrrolidin-1-ylcarbonyl group);
$R^{31}$ is a halogen atom, an amino group, a C1-C6 alkoxy group, or a $-CONH_2$ group; and
$R^4$ represents a hydrogen atom.

An example of a particularly preferable sulfonamide compound is as follows.

5-Chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide ("Compound 1")

Aspects of the present disclosure are as described below.

- A pharmaceutical composition for treating and/or preventing a tumor comprising Compound 1 or a salt thereof in combination with an immune checkpoint molecule regulator.
- Compound 1 or a salt thereof for use in the treatment and/or the prevention of a tumor, which is used in combination with an immune checkpoint molecule regulator.
- A combination of an immune checkpoint molecule regulator and Compound 1 or a salt thereof used for treating

and/or preventing a tumor.

- An agent for enhancing antitumor effects of an immune checkpoint molecule regulator comprising Compound 1 or a salt thereof.

The sulfonamide compound represented by Formula (I) of the present disclosure or a salt thereof can be prepared in accordance with the method described in, for example, WO 2017/209155.

The compound represented by Formula (I) of the present disclosure and intermediates thereof can be isolated and purified by known separation and purification means such as recrystallization, crystallization, distillation, or column chromatography. The sulfonamide compound represented by Formula (I) and synthetic intermediates thereof can form a pharmacologically acceptable salt thereof in accordance with a conventional technique, and they can be converted to each other.

When optical isomers, stereoisomers, tautomers, or rotary isomers are present in the sulfonamide compound represented by Formula (I), the sulfonamide compound represented by Formula (I) encompasses these isomers or the mixture thereof. When optical isomers are present in the sulfonamide compound represented by Formula (I), for example, a racemate and an optical isomer resolved from a racemate are also included in the sulfonamide compound represented by Formula (I), unless otherwise stated. Each of these isomers can be obtained by known synthetic and separation means (e.g., concentration, solvent extraction, column chromatography, or recrystallization) as a single compound. For example, in the sulfonamide compound represented by Formula (I), in which $X^1$ represents an oxygen atom, $X^2$ represents an oxygen atom, and $X^3$ represents NH, tautomers as shown below are present, and any of the isomers are within the scope of the present disclosure.

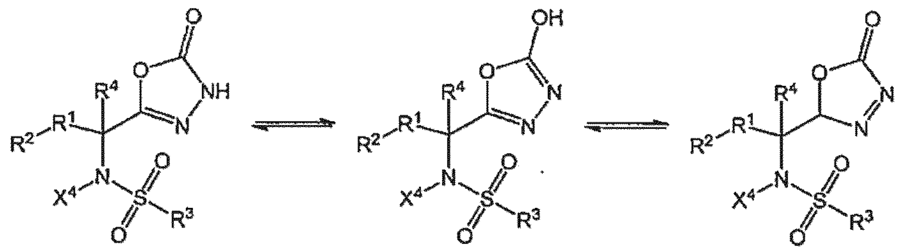

The sulfonamide compound represented by Formula (I) or a salt thereof may be amorphous or in a crystalline form, and the crystalline form may be a single crystalline form or a polymorphic mixture. Crystals can be prepared by known crystallization methods. Cocrystals formed by the sulfonamide compound represented by Formula (I) or a salt thereof and other components are encompassed in the crystals. Furthermore, the sulfonamide compound represented by Formula (I) or a salt thereof can be a solvate (e.g., a hydrate) or a non-solvate, and the both thereof are encompassed in the sulfonamide compound represented by Formula (I) or a salt thereof. Compounds labeled with isotopes (e.g., deuterium, $^{3}H$, $^{14}C$, $^{35}S$, and $^{125}I$) are also encompassed in the sulfonamide compound represented by Formula (I) or a salt thereof.

While prodrugs of the sulfonamide compound represented by Formula (I) or a salt thereof are also encompassed in the present disclosure, the prodrugs refer to, for example, compounds which are converted into the sulfonamide compound represented by Formula (I) or a salt thereof by a reaction with an enzyme or gastric acid under the physiological condition in the living body, i.e., compounds which are converted into the sulfonamide compound represented by Formula (I) or a salt thereof by enzymatic oxidation, reduction, or hydrolysis or compounds which are converted into the sulfonamide compound represented by Formula (I) or a salt thereof by hydrolysis caused by gastric acid. Furthermore, prodrugs of the sulfonamide compound represented by Formula (I) or a salt thereof may be the compounds which are converted into the sulfonamide compound represented by Formula (I) or a salt thereof under physiological conditions as described in Hirokawa Shoten 1990 annual, "Development of Pharmaceuticals," Volume 7, Molecular Design, pp. 163-198.

The salt of the sulfonamide compound represented by Formula (I) is a pharmaceutically acceptable salt.

The sulfonamide compound represented by Formula (I) or a salt thereof has an inhibitory activity against RNR. The sulfonamide compound represented by Formula (I) or a salt thereof is useful as a medicament for prevention or treatment of RNR-related diseases without causing side effects based on the off-target effects on iron ion-requiring protein, due to its excellent RNR inhibitory activity and its structure that does not chelate to metal ions.

Use of the sulfonamide compound represented by Formula (I) or a salt thereof in combination with the immune checkpoint molecule regulator can enhance their antitumor effects.

The immune checkpoint molecule regulator of the present disclosure directly acts on an immune checkpoint molecule to induce antitumor immune responses in the body of a cancer patient and regulates tumor immune evasion.

Examples thereof include substances that accelerate the functions of costimulatory molecules and substances that inhibit the functions of coinhibitory molecules. Examples of immune checkpoint molecules include B7 family (e.g.,

B7-1, B7-2, PD-L1, and PD-L2) molecules, CD28 family (e.g., CTLA-4 and PD-1) molecules, TNF superfamily (4-1BBL and OX40L) molecules, and TNF receptor superfamily (4-1BB and OX40) molecules. As immune checkpoint molecule regulators, substances targeting such immune checkpoint molecules can be used. Examples thereof include a PD-1 pathway antagonist, an ICOS pathway agonist, a CTLA-4 pathway antagonist, a CD28 pathway agonist, a BTLA pathway antagonist, and a 4-1BB pathway agonist.

In the present disclosure, the immune checkpoint molecule regulator is preferably at least 1 member selected from among a PD-1 pathway antagonist, an ICOS pathway agonist, a CTLA-4 pathway antagonist, and a CD28 pathway agonist, more preferably at least 1 member selected from among a PD-1 pathway antagonist and a CTLA-4 pathway antagonist, and further preferably a PD-1 pathway antagonist, from the viewpoint of suppression of side effects.

The PD-1 pathway antagonist inhibits the immunosuppressive signals of PD-1 expressed on T cells or a ligand thereof; i.e., PD-L1 or PD-L2. Examples thereof include an anti-PD-1 antibody, an anti-PD-Ll antibody, an anti-PD-L2 antibody, a PD-1 extracellular domain, a PD-L1 extracellular domain, a PD-L2 extracellular domain, PD-1-Ig (a fusion protein of a PD-1 extracellular domain and the FC region of immunoglobulin (Ig)), PD-L1-Ig, PD-L2-Ig, PD-1 siRNA, PD-L1 siRNA, and PD-L2 siRNA, with the anti-PD-1 antibody, the anti-PD-L1 antibody, or the anti-PD-L2 antibody being preferable, the anti-PD-1 antibody or the anti-PD-L1 antibody being more preferable, and the anti-PD-1 antibody being particularly preferable.

The CTLA-4 pathway antagonist inhibits the immunosuppressive signals of CTLA-4 expressed on T cells or a ligand thereof; i.e., B7-1 (CD80) or B7-2 (CD86). The CTLA-4 pathway antagonist is preferably an anti-CTLA-4 antibody, a CTLA-4 extracellular domain, CTLA-4-Ig, an anti-B7-1 (CD80) antibody, or an anti-B7-2 (CD86) antibody, more preferably the anti-CTLA-4 antibody or CTLA-4-Ig, and particularly preferably the anti-CTLA-4 antibody.

According to an embodiment of the present disclosure, the immune checkpoint molecule regulator is preferably at least 1 member selected from among the anti-PD-1 antibody, the anti-PD-Ll antibody, and the anti-CTLA-4 antibody.

Examples of such antibodies include immunoglobulins (e.g., IgA, IgD, IgE, IgG, IgM, and IgY), Fab fragments, F(ab')2 fragments, single-chain antibody fragments (scFv), single domain antibodies, and diabodies (Nat. Rev. Immunol., 6: 343-357, 2006). Examples of antibodies include human, humanized, chimeric, mouse, llama, and chicken monoclonal and polyclonal antibodies.

Preferable antibodies are humanized IgG monoclonal antibodies or human IgG monoclonal antibodies.

Preferable examples of the anti-PD-1 antibodies according to the present disclosure include Nivolumab and Pembrolizumab.

Preferable examples of the anti-PD-Ll antibodies according to the present disclosure include Atezolizumab, Durvalumab, and Avelumab, with Atezolizumab being more preferable.

Examples of the anti-CTLA-4 antibodies according to the present disclosure include Ipilimumab and Tremelimumab, with Ipilimumab being preferable.

A preferable example of CTLA-4-Ig according to the present disclosure is Abatacept.

Such antibodies can be produced in accordance with a conventional method of antibody production.

The anti-PD-1 antibody is already sold or scheduled to be sold as Nivolumab or Pembrolizumab, the anti-PD-Ll antibody is already sold or scheduled to be sold as Atezolizumab, Durvalumab, or Avelumab, the anti-CTLA-4 antibody is already sold or scheduled to be sold as Ipilimumab or Tremelimumab, and CTLA-4-Ig is already sold or scheduled to be sold as Abatacept, and such antibodies can be used.

When 2 or more types of immune checkpoint molecule regulators are used according to the present disclosure, for example, the anti-PD-1 antibody may be used in combination with the anti-CTLA-4 antibody, or a bispecific antibody that can bind to both PD-1 and CTLA-4 may be used. An example of a bispecific antibody is XmAb20717 (PD-1 × CTLA-4).

In an embodiment of the present disclosure, a daily dose of the sulfonamide compound represented by Formula (I) or a salt thereof on the day of administration can be adequately determined with reference to, for example, a dose of the sulfonamide compound represented by Formula (I) or a salt thereof when it is administered alone. In one aspect, the daily dose is 50% to 200% of the dose administered alone. In another aspect, the daily dose is 75% to 150% of the dose administered alone. In another aspect, the a daily dose is 87.5% to 112.5% of the dose administered alone. In another aspect, the daily dose is 100% of the dose administered alone.

In the present disclosure, a dose of the sulfonamide compound represented by Formula (I) or a salt thereof to a human on the day of administration is, in one aspect, 1 $mg/m^2$/day to 10 $mg/m^2$/day in the form of the sulfonamide compound. In another aspect, the dose is 10 $mg/m^2$/day to 100 $mg/m^2$/day. In another aspect, the dose is 100 $mg/m^2$/day to 1,000 $mg/m^2$/day. In another aspect, the dose is 1,000 $mg/m^2$/day to 10,000 $mg/m^2$/day.

In the present disclosure, the daily dose of the immune checkpoint molecule regulator on the day of administration is preferably 50% to 100%, and more preferably 100%, relative to the recommended dose of the immune checkpoint molecule regulator when it is administered alone.

Specifically, the recommended dose of Nivolumab when administered alone is 2 mg/kg (body weight) or 3 mg/kg (body weight) per instance, which is the dose approved in Japan. In the present disclosure, accordingly, a daily dose of Nivolumab is preferably 1 to 3 mg/kg (body weight), and more preferably 2 mg/kg (body weight) or 3 mg/kg

(body weight), per instance, on the day of administration.

The recommended dose of Pembrolizumab when administered alone is 2 mg/kg (body weight) or 200 mg per instance, which is the dose approved in Japan. In the present disclosure, accordingly, a daily dose of Pembrolizumab is preferably 1 to 2 mg/kg (body weight) or 100 to 200 mg, and more preferably 2 mg/kg (body weight) or 200 mg, per instance, on the day of administration.

The recommended dose of Atezolizumab when administered alone is 1,200 mg per instance, which is the dose approved in U.S.A. In the present disclosure, accordingly, a daily dose of Atezolizumab is preferably 600 to 1,200 mg, and more preferably 1,200 mg, per instance, on the day of administration.

The recommended dose of Avelumab or Durvalumab when administered alone is 10 mg/kg (body weight) per instance, which is the dose approved in U.S.A. In the present disclosure, accordingly, a daily dose of Avelumab or Durvalumab is preferably 5 to 10 mg/kg (body weight), and more preferably 10 mg/kg (body weight), per instance, on the day of administration.

The recommended dose of Ipilimumab when administered alone is 3 mg/kg (body weight) per instance, which is the dose approved in Japan. In the present disclosure, accordingly, a daily dose of Ipilimumab is preferably 1.5 to 3 mg/kg (body weight), and more preferably 3 mg/kg (body weight), per instance, on the day of administration.

The term "recommended dose" used in the present disclosure refers to a dose determined on the basis of clinical trials and the like at which a medicament of interest can be used safely without developing any serious side effects and can exert maximal therapeutic effects. Specifically, the "recommended dose" is approved, recommended, and/or advised by public organizations or institutions, such as the Pharmaceuticals and Medical Devices Agency (PMDA), Japan, the Food and Drug Administration (FDA), U.S.A., or the European Medicines Agency (EMA), and described in, for example, the product information, the interview form, or treatment guidelines. The "recommended dose" is preferably the dose approved by any of PMDA, FDA, or EMA.

The administration schedule of the antitumor agent according to the present disclosure can be adequately determined in accordance with, for example, a cancer type or a disease stage.

In the case of Nivolumab, administration is preferably performed at intervals of 2 or 3 weeks.

In the case of Pembrolizumab, Atezolizumab, or Ipilimumab, administration is preferably performed at intervals of 3 weeks.

The frequency of administration of the antitumor agent per day according to the present disclosure can be adequately determined in accordance with, for example, a cancer type or a disease stage.

In the case of the sulfonamide compound represented by Formula (I) or a salt thereof, administration is preferably performed once or twice a day, and more preferably once a day. The administration of Nivolumab, Pembrolizumab, Atezolizumab, or Ipilimumab is preferably performed once a day.

The order for administration of the sulfonamide compound represented by Formula (I) or a salt thereof and the immune checkpoint molecule regulator according to the present disclosure can be adequately determined in accordance with, for example, a cancer type or a disease stage. The order for administration is not particularly limited, and both the agents may be administered simultaneously. When both the agents are not administered simultaneously, the intervals for administration should be adequately determined within a period during which the effects of enhancing antitumor effects can be exerted. The intervals are preferably 1 to 14 days, more preferably 1 to 7 days, further preferably 1 to 5 days, and particularly preferably 1 to 3 days.

A combination formulation of the sulfonamide compound represented by Formula (I) or a salt thereof and the immune checkpoint molecule regulator may be in the form of a single formulation (i.e., a blended formulation) or in the form of 2 or more separate formulations to be administered in combination.

In the present disclosure, the antitumor effect can be evaluated on the basis of, for example, decrease in tumor volume, stagnant tumor growth, or prolongation of survival periods.

In an embodiment, an antitumor agent comprising the sulfonamide compound represented by Formula (I) or a salt thereof and the immune checkpoint molecule regulator in combination is provided. In another embodiment, an agent for enhancing antitumor effects of the immune checkpoint molecule regulator comprising, as an active ingredient, the sulfonamide compound represented by Formula (I) or a salt thereof is provided.

In the present disclosure, "cancer" or "tumor" indicates a physiological state of a mammalian animal characterized by disorganized cell growth. The terms "cancer" and "tumor" are used herein in the same sense, and used interchangeably with each other. Cancer encompasses solid cancer and blood cancer. Examples thereof include, but are not limited to, carcinoma, lymphoma, leukemia, blastoma, sarcoma, and borderline malignant tumor (carcinoid).

Tumors of interest in the present disclosure are not particularly limited, as long as the effect of enhancing an antitumor effect can be exerted. A tumor on which the sulfonamide compound represented by Formula (I) or a salt thereof can exert an antitumor effect is preferred, and an RNR-related malignant tumor is more preferred.

Examples of "RNR-related malignant tumors" includes malignant tumors in which the incidence can be lowered, the symptoms can be resolved, alleviated, and/or completely cured by deleting, suppressing, and/or inhibiting RNR functions. Examples of malignant tumors to be treated include, but are not particularly limited to, head and neck cancer,

gastrointestinal cancer (esophageal cancer, gastric cancer, duodenal cancer, liver cancer, biliary tract cancer (gallbladder/bile duct cancer), pancreatic cancer, and colorectal cancer (colon cancer and rectal cancer)), lung cancer (non-small cell lung cancer, small cell lung cancer, and mesothelioma), breast cancer, genital cancer (ovarian cancer and uterine cancer (cervical cancer and endometrial cancer)), urinary organ cancer (renal cancer, bladder cancer, prostate cancer, and testicular tumor), hematopoietic tumors (leukemia, malignant lymphoma, and multiple myeloma), bone and soft tissue tumors, skin cancer, and brain tumor.

Examples of target malignant tumors include, but are not particularly limited to, adenocarcinoma, carcinoid, undifferentiated carcinoma, angiosarcoma, adenocarcinoma, gastrointestinal cancer (colorectal cancer (CRC) including colon cancer and rectal cancer, biliary tract cancer including gallbladder cancer and bile duct cancer, anal cancer, esophageal cancer, gastric cancer, gastrointestinal carcinoid tumor, gastrointestinal stromal tumor (GIST), hepatic cancer, duodenal cancer, and small intestinal cancer), lung cancer (non-small cell lung cancer (NSCLC), squamous lung cancer, large cell lung cancer, small cell lung cancer, mesothelial tumor, and other lung cancer such as bronchial tumor and pleuropulmonary blastoma), urinary organ cancer (renal cancer, transitional cell cancer of the kidney (TCC), TCC of the renal pelvis and the urinary tract (PDQ), bladder cancer, urethral cancer, and prostate cancer), head and neck cancer (ocular cancer, glioma retinae, intraocular melanoma, hypopharyngeal cancer, pharyngeal cancer, laryngeal cancer, laryngeal papillomatosis, metastatic squamous cell cancer of the neck of unknown primary, intraoral cancer, labial cancer, throat cancer, oropharynx cancer, sensory neuroblastoma, nasal cancer and paranasal cancer, nasopharyngeal cancer, and salivary gland cancer), endocrine cancer (thyroid gland cancer, parathyroid gland cancer, multiple endocrine neoplasia syndromes, thymic tumor and thymic cancer, pancreatic cancer including pancreatic ductal adenocarcinoma (PDAC), pancreatic neuroendocrine tumor, and pancreatic islet cell tumor), breast cancer (ductal cancinoma *in situ* (DCIS), lobular carcinoma *in situ* (LCIS), triple-negative breast cancer, and inflammatory breast cancer), cancer of male and female reproductive organs (cervical cancer, ovarian cancer, endometrial cancer, uterine sarcoma, uterine cancer, vaginal cancer, vulvar cancer, gestational trophoblastic tumor (GTD), extragonadal germ cell tumor, extracranial germ cell tumor, germ cell tumor, testicular carcinoma, and penile cancer), brain and nervous system cancer (astroglioma, brain stem glioma, brain tumor, craniopharyngioma, central nervous system (CNS) cancer, chordoma, ependymoma, embryonal tumor, neuroblastoma, paraganglioma, and atypical teratoid tumor), skin cancer (basal cell cancer (BCC), squamous cell cancer (SCC), Merkel cell cancer, and melanoma), tissue and bone cancer (soft tissue sarcoma, rhabdomyosarcoma, fibrous histiocytoma of bone, Ewing's sarcoma, malignant fibrous histiocytoma (MFH) of bone, osteosarcoma, and chondrosarcoma), cardiovascular cancer (cardiac cancer and cardiac tumor), appendiceal cancer, pediatric and adolescent cancer (pediatric adrenocortical cancer, midline tract cancer, hepatic cell cancer (HCC), congenital hepatoma, and Wilms tumor), and virus-induced cancer (HHV-8-associated cancer (Kaposi's sarcoma) and HIV/AIDS-associated cancer).

Examples of target malignant tumors include, but are not particularly limited to, hematological disorders and malignant plasma cell tumors, such as multiple myeloma, leukemia, lymphoma, myelodysplastic syndromes, and myeloproliferative disorders (i.e., cancer that affects blood, bone marrow, and/or lymph node). Examples of leukemia include, but are not limited to, acute lymphoblastic leukemia (ALL), acute myelocytic leukemia (AML), chronic lymphatic leukemia (CLL), chronic myelocytic leukemia (CML), acute monocytic leukemia (AMoL), hairy cell leukemia, and/or other leukemic diseases. Examples of lymphoma include, but are not limited to, Hodgkin's lymphoma and non-Hodgkin's lymphoma (NHL). In one aspect, NHL is B-cell lymphoma and/or T-cell lymphoma. In some embodiments, examples of NHL include, but are not limited to, diffuse large B-cell lymphoma (DLBCL), small lymphocytic lymphoma (SLL), chronic lymphocytic leukemia (CLL), mantle cell lymphoma (MCL), Burkitt's lymphoma, cutaneous T-cell lymphoma including mycosis fungoides lymphoma and Sezary syndrome, AIDS-associated lymphoma, follicular lymphoma, lymphoplasmacytic lymphoma (Waldenstrom's macroglobulinaemia (WM)), primary central nervous system (CNS) lymphoma, and/or other lymphomas.

The antitumor agent of the present invention may be used for postoperative adjuvant chemotherapy performed for the purpose of recurrence prevention after surgical tumor removal, or for preoperative adjuvant chemotherapy performed before surgical tumor removal.

"RNR" used herein indicates a human or non-human RNR, with the human RNR being preferable.

When using the sulfonamide compound represented by Formula (I) or a salt thereof and the immune checkpoint molecule regulator as a medicament, it is optionally formulated with a pharmaceutically acceptable carrier, and various dosage forms can be adopted depending on the prevention or therapeutic purposes, and the dosage forms may be, for example, any of oral agents, injections, suppositories, ointments, and patches. Since the sulfonamide compound represented by Formula (I) or a salt thereof has an excellent oral absorbability, oral agents are preferable. These dosage forms can be prepared by conventional preparation methods known to a person skilled in the art.

With respect to pharmaceutically acceptable carriers, various conventional organic or inorganic carrier substances are used as pharmaceutical materials, and formulated as: excipients, binders, disintegrators, lubricants, or coloring agents for solid formulations; and solvents, solubilizing agents, suspending agents, isotonic agents, buffers, or soothing agents for liquid formulations, and the like. If desired, pharmaceutical additives, such as preservative agents,

antioxidants, coloring agents, sweetening agents, flavoring agents, or stabilizing agents, can also be used.

In general, examples of the pharmaceutically acceptable carriers and the pharmaceutical additives include: as the excipient, lactose, sucrose, sodium chloride, glucose, starch, calcium carbonate, kaolin, microcrystalline cellulose, and silicic acid; as binders, water, ethanol, propanol, simple syrup, a glucose solution, a starch solution, a gelatin solution, carboxymethyl cellulose, hydroxypropyl cellulose, hydroxypropyl starch, methyl cellulose, ethyl cellulose, shellac, calcium phosphate, and polyvinylpyrrolidone; as disintegrators, dry starch, sodium alginate, agar powder, sodium hydrogen carbonate, calcium carbonate, sodium lauryl sulfate, stearic acid monoglyceride, and lactose; as lubricants, purified talc, stearate, borax, and polyethylene glycol; as coloring agents, titanium oxide and iron oxide; and, as flavoring agents, sucrose, orange peel, citric acid, and tartaric acid.

An oral solid formulation can be prepared by adding an excipient to the sulfonamide compound represented by Formula (I) or the immune checkpoint molecule regulator, further adding, if necessary, binders, disintegrators, lubricants, coloring agents, and/or flavoring agents thereto, and formulating into tablets, coated tablets, granules, powders, capsules, or the like.

An injection can be prepared by adding pH control agents, buffers, stabilizers, isotonic agents, local anesthetic agents, and the like to the sulfonamide compound represented by Formula (I) or the immune checkpoint molecule regulator, followed by formulating into subcutaneous, intramuscular, or intravenous injections in accordance with a conventional technique.

The amount of the sulfonamide compound represented by Formula (I) to be formulated in each of the dosage unit forms described above varies depending on, for example, the symptoms of the patients to which the compound is administered or dosage forms. In general, it is preferable that 0.05 to 1,000 mg thereof be formulated in an oral preparation, about 0.01 to 500 mg thereof be formulated in an injection preparation, and 1 to 1000 mg thereof be formulated in a suppository, per one dosage unit form.

The administration schedule of the sulfonamide compound represented by Formula (I) or a salt thereof and the immune checkpoint molecule regulator is adequately determined within a range in which each active ingredient exerts an antitumor effect, and the active ingredients are administered concurrently or separately in a staggered manner. For separate administration, either of the active ingredients may be administered first.

The sulfonamide compound represented by Formula (I) or a salt thereof and the immune checkpoint molecule regulator may be formulated into 2 or more dosage forms each containing a relevant active ingredient or may be formulated collectively into one dosage form, depending on the dosage form or the schedule for administration of each active ingredient. Further, the formulations may be manufactured and distributed in a package suitable for combined use or may be manufactured and distributed in separate packages.

The present invention also relates to a kit formulation comprising an antitumor agent containing the sulfonamide compound represented by Formula (I) or a salt thereof and instructions for use describing that the sulfonamide compound represented by Formula (I) or a salt thereof is administered to a cancer patient in combination with an immune checkpoint molecule regulator.

It is sufficient if "instructions for use" contain a description concerning the dose described above. It is preferable that the dose is the recommend dose described above, regardless of legally binding force. Specific examples of the instructions for use include the product information and the pamphlet. The kit formulation comprising the instructions for use may include the instructions for use printed and/or attached to a package of the kit formulation, or the kit formulation may contain the antitumor agent and the instructions for use in the package thereof.

## Examples

Hereafter, the present invention is described in greater detail with reference to the examples, although the present invention is not limited to these examples. A person skilled in the art would be able to make various forms of modification within the technical scope of the present invention.

[Example 1: Effects of Compound 1 in combination with anti-mouse PD-1 antibody on colon cancer cell-transplanted models]

The mouse colon cancer cell strain MC38 was obtained from Dr. Yoshihiro Hayakawa (University of Toyama, Toyama, Japan). The MC38 cell strain was subjected to culture in an RPMI 1640 medium containing 10% fetal bovine serum and then to subculture in an incubator at 37°C in the presence of 5% $CO_2$ at a frequency of once or twice a week.

The MC38 cell suspension was transplanted subcutaneously at $1 \times 10^6$ cells/0.1 ml to the right chest of 6-week-old C57BL/6NCrl mice (Charles River Laboratories Japan, Inc.).

After transplantation, tumors were allowed to grow to reach the tumor volume (TV) of 50 to 300 $mm^3$. The longer diameter and the shorter diameter of the tumor were measured using a Digimatic caliper and the tumor volume (TV) was calculated in accordance with the following formula.

$$TV\ (mm^3) = \text{longer diameter (mm)} \times \text{shorter diameter (mm)} \times \text{shorter diameter (mm)}/2$$

The animals were divided into groups each consisting of 10 individuals by stratified random allocation using TV as the indicator. The day on which grouping (n = 10) was performed was designated as Day 0.
Tumor volume changes (T/C) were calculated based on TV on the final day of evaluation (Day 15).

$$T/C\ (\%) = \text{(average TV of each drug administration group)/(average TV of control group)} \times 100$$

Body weights were measured using an electronic balance for animals. Body weight changes (BWCn) on the $n^{th}$ day were determined based on the body weight measured on the $n^{th}$ day (BWn) in accordance with the following formula.

$$BWCn\ (\%) = (BWn - BW0)/BW0 \times 100$$

An aqueous solution of 0.5% Hypromellose was prepared by adding an adequate amount of an injection solvent (Japanese Pharmacopoeia) to and dissolving Hypromellose at a concentration of 0.5 w/v%. Compound 1, synthesized in accordance with the method disclosed in WO 2017/209155, was grounded in an agate mortar, suspended at a given concentration in the aqueous solution of 0.5% Hypromellose, and then ultrasonically treated to obtain a homogeneous suspension. The resulting suspension was orally administered as Compound 1 once a day for continuous 14 days at 25 mg/kg/day or 50 mg/kg/day. To the control group, the aqueous solution of 0.5% Hypromellose was orally administered once a day for continuous 14 days.
As an anti-mouse PD-1 antibody (anti-mPD-1 Ab), CD279 (PD-1) Monoclonal Antibody (RMP1-14), eBioscience (Thermo Fisher Scientific) was diluted to a given concentration with PBS immediately before administration. On the first day of administration (Day 1), the anti-mouse PD-1 antibody was administered intraperitoneally at 0.05 mg/body.
The results are shown in Table 1 and Figs. 1 to 4.

[Table 1]

| Group | Dose (/day) | Schedule (day) | Route | TV , on Day 15 ($mm^3$, mean ± SE) | T/C (%) | BWC on Day 15 (%, meair±SE) |
|---|---|---|---|---|---|---|
| **Control** | **-** | **1-14** | p.o. | 998 ± 110 | - | 3.5 ± 0.4 |
| **Anti-mPD-1 Ab** | **0.05 mg/ body** | **1** | i.p. | 436 ± 103 | 44.4 | 9.7 ±0.7 |
| **Compound 1** | **25 mg/kg** | **1-14** | p.o. | 868 ± 182 | 91.9 | 4.0 ± 0.7 |
| **Anti-mPD-1 Ab + Compound 1** | **0.05 mg/ body + 25 mg/kg** | **1 + 1-14** | i.p. ± p.o. | 274 ± 107 | 26.6 | 4.1 ± 1.0 |
| **Compound 1** | **50 mg/kg** | **1-14** | p.o. | 306 ± 79 | 29.7 | 4.9 ± 1.0 |
| **Anti-mPD-1 Ab + Compound 1** | **0.05 mg/ body + 50 mg/kg** | **1 +1-14** | i.p. + p.o. | 92.4 ± 37.6 | 8.67 | 2.5 ± 1.1 |

*$p < 0.05$ Dunnett's test as compared with the control group.
# : $p < 0.05$ Student's t-test as compared with the anti-mouse PD-1 antibody (anti-mPD-1 Ab) group.
S : $p < 0.05$ Student's *t*-test as compared with the Compound 1 group.
SE: standard error

As a result of analysis of TV of each group on Day 15 by the Dunnett' test, TVs measured in the group to which Compound 1 had been administered at 50 mg/kg/day, the group to which the anti-mouse PD-1 antibody had been administered alone, and the group to which Compound 1 and the anti-mouse PD-1 antibody had been administered in combination were found to be significantly lower than those measured in the control group, and antitumor effects were

demonstrated. As a result of analysis of TV on Day 15 by the Student' t-test, TV measured in the group to which Compound 1 (50 mg/kg/day) and the anti-mouse PD-1 antibody had been administered in combination was found to be significantly lower than TV measured in the group to which Compound 1 had been administered alone at 50 mg/kg/day or the group to which the anti-mouse PD-1 antibody had been administered alone, demonstrating higher antitumor effects.

In contrast, average body weight changes in the group to which Compound 1 and the anti-mouse PD-1 antibody had been administered in combination were not associated with enhanced toxicity, compared with the groups to which Compound 1 or the anti-mouse PD-1 antibody had been administered alone.

All publications, patents, and patent applications cited herein are incorporated herein by reference in their entirety.

## Claims

1. A pharmaceutical composition for treating and/or preventing a tumor comprising a sulfonamide compound represented by Formula (I) below or a salt thereof:

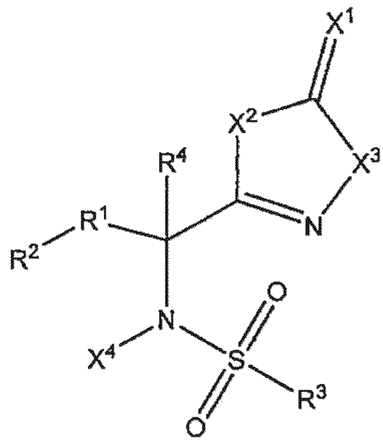

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom,

wherein the compound or the salt thereof is used in combination with an immune checkpoint molecule regulator.

**2.** The pharmaceutical composition according to Claim 1, wherein, in Formula (I):

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
$R^{11}$ and $R^{12}$ are the same or different and represent a hydrogen atom or a C1-C6 alkyl group;
$R^2$ represents a C6-C14 aromatic hydrocarbon group, and $R^2$ may have $R^{21}$ as a substituent;
$R^{21}$ represents a halogen atom or a C1-C6 alkyl group (when 2 or more $R^{21}$ are present, $R^{21}$ may be the same with or different from each other);
$R^3$ represents a C6-C14 aromatic hydrocarbon group which may have $R^{31}$ as a substituent or may be fused with a 4- to 8-membered saturated heterocyclic ring (wherein the saturated heterocyclic ring may have Rc as a substituent);
$R^{31}$ represents a halogen atom or an aminocarbonyl group (when 2 or more $R^{31}$ are present, $R^{31}$ may be the same with or different from each other);
Rc represents a halogen atom, a hydroxy group, or a C1-C6 alkyl group (when 2 or more Rc are present, Rc may be the same with or different from each other); and
$R^4$ represents a hydrogen atom.

**3.** The pharmaceutical composition according to Claim 1 or Claim 2, wherein, in Formula (I),

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
either $R^{11}$ or $R^{12}$ represents a hydrogen atom, and the other represents a C1-C6 alkyl group;
$R^2$ represents a phenyl group, and $R^2$ may have $R^{21}$ as a substituent;
$R^{21}$ represents a halogen atom or a C1-C6 alkyl group (when 2 or more $R^{21}$ are present, $R^{21}$ may be the same with or different from each other);
$R^3$ represents a phenyl group which may have $R^{31}$ as a substituent or may be fused with a monocyclic 6-membered saturated heterocyclic ring having 1 oxygen atom (wherein the saturated heterocyclic ring may have Rc as a substituent);
$R^{31}$ represents a halogen atom or an aminocarbonyl group (when 2 or more $R^{31}$ are present, $R^{31}$ may be the same with or different from each other);
Rc represents a halogen atom, a hydroxy group, or a C1-C6 alkyl group (when 2 or more Rc are present, Rc may be the same with or different from each other); and
$R^4$ represents a hydrogen atom.

**4.** The pharmaceutical composition according to any one of Claims 1 to 3, wherein, in Formula (I):

$X^1$ represents an oxygen atom;
$X^2$ represents an oxygen atom;
$X^3$ represents -NH-;
$X^4$ represents a hydrogen atom;
$R^1$ represents $-C(R^{11})(R^{12})-$;
either $R^{11}$ or $R^{12}$ represents a hydrogen atom, and the other represents a methyl group;
$R^2$ represents a phenyl group having $R^{21}$ as a substituent;
$R^{21}$ represents a halogen atom or a C1-C6 alkyl group (when 2 or more $R^{21}$ are present, $R^{21}$ may be the same with or different from each other);
$R^3$ represents a phenyl group having $R^{31}$ as a substituent or a chromanyl group having Rc as a substituent;
$R^{31}$ represents a halogen atom or an aminocarbonyl group (when 2 or more $R^{31}$ are present, $R^{31}$ may be the same with or different from each other);
Rc represents a halogen atom, a hydroxy group, or a C1-C6 alkyl group (when 2 or more Rc are present, Rc may be the same with or different from each other); and
$R^4$ represents a hydrogen atom.

**5.** The pharmaceutical composition according to any one of Claims 1 to 4, wherein the sulfonamide compound is 5-chloro-2-(N-((1S,2R)-2-(6-fluoro-2,3-dimethylphenyl)-1-(5-oxo-4,5-dihydro-1,3,4-oxadiazol-2-yl)propyl)sulfamoyl)benzamide.

**6.** The pharmaceutical composition according to any one of Claims 1 to 5, wherein a sulfonamide compound represented by Formula (I) or a salt thereof and an immune checkpoint molecule regulator are administered concurrently, sequentially, or in a staggered manner.

**7.** The pharmaceutical composition according to any one of Claims 1 to 6, wherein the immune checkpoint molecule regulator is at least 1 member selected from among a PD-1 pathway antagonist, an ICOS pathway agonist, a CTLA-4 pathway antagonist, and a CD28 pathway agonist.

**8.** The pharmaceutical composition according to any one of Claims 1 to 7, wherein the immune checkpoint molecule regulator is a PD-1 pathway antagonist.

**9.** The pharmaceutical composition according to Claim 8, wherein the PD-1 pathway antagonist is at least 1 member selected from the group consisting of an anti-PD-1 antibody, an anti-PD-L1 antibody, and an anti-PD-L2 antibody.

**10.** The pharmaceutical composition according to Claim 8, wherein the PD-1 pathway antagonist is an anti-PD-1 antibody.

**11.** The pharmaceutical composition according to Claim 10, wherein the anti-PD-1 antibody is Nivolumab or Pembrolizumab.

**12.** A sulfonamide compound represented by Formula (I) or a salt thereof for use in the treatment and/or the prevention of a tumor, which is used in combination with an immune checkpoint molecule regulator:

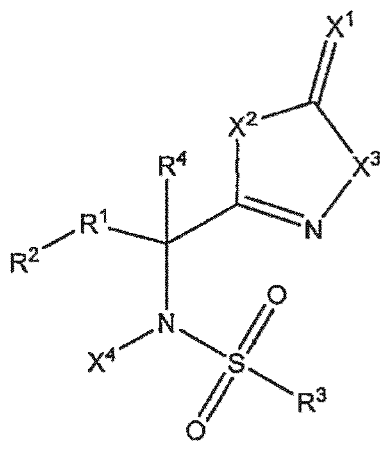

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic

group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and

$R^4$ represents a hydrogen atom or a C1-C6 alkyl group,

provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

**13.** Use of a sulfonamide compound represented by Formula (I) or a salt thereof for producing a medicament used for the treatment and/or the prevention of a tumor, which is used in combination with an immune checkpoint molecule regulator:

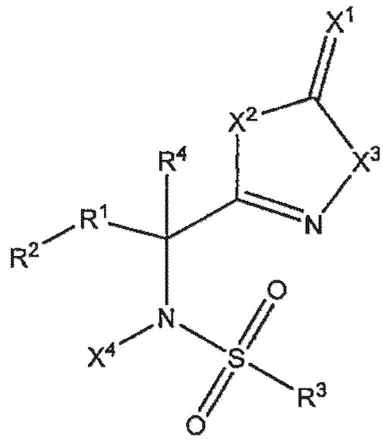

(I)

wherein,

$X^1$ represents an oxygen atom or a sulfur atom;

$X^2$ represents an oxygen atom or -NH-;

$X^3$ represents -NH- or an oxygen atom;

$X^4$ represents a hydrogen atom or a C1-C6 alkyl group;

$R^1$ represents $-C(R^{11})(R^{12})-$ or $-C(=CH_2)-$;

$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;

$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;

$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and

$R^4$ represents a hydrogen atom or a C1-C6 alkyl group,

provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

**14.** A method for treating and/or preventing a tumor comprising administering an effective amount of a sulfonamide compound represented by Formula (I) or a salt thereof to a patient in combination with an immune checkpoint molecule regulator:

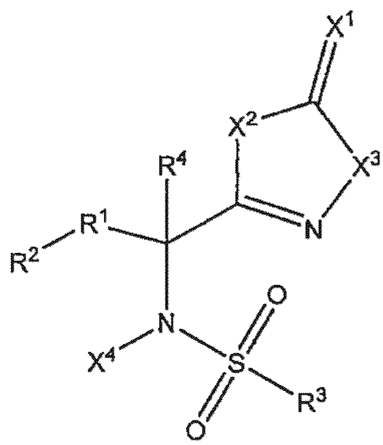

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

**15.** A combination of an immune checkpoint molecule regulator and a sulfonamide compound represented by Formula (I) or a salt thereof used for treating and/or preventing a tumor:

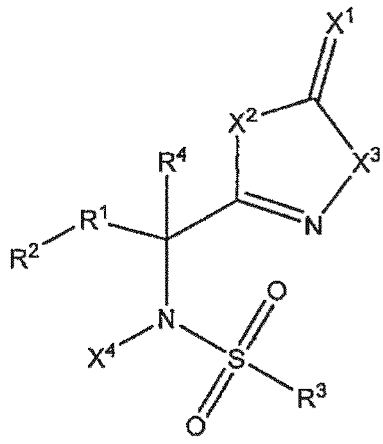

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group;
$R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

**16.** An agent for enhancing antitumor effects of an immune checkpoint molecule regulator comprising a sulfonamide compound represented by Formula (I) or a salt thereof:

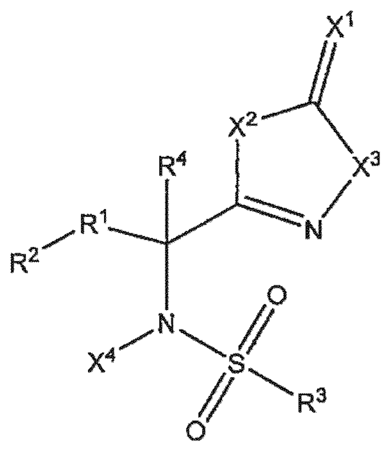

(I)

wherein,

$X^1$ represents an oxygen atom or a sulfur atom;
$X^2$ represents an oxygen atom or -NH-;
$X^3$ represents -NH- or an oxygen atom;
$X^4$ represents a hydrogen atom or a C1-C6 alkyl group; $R^1$ represents $-C(R^{11})(R^{12})-$ or $-C(=CH_2)-$;
$R^{11}$ and $R^{12}$ may be the same or different, represent a hydrogen atom, a halogen atom, a hydroxy group, or a C1-C6 alkyl group, or form, together with the carbon atoms to which they bind, a saturated hydrocarbon ring having 3 to 8 carbon atoms;
$R^2$ represents a C6-C14 aromatic hydrocarbon group or a 9- or 10-membered fully unsaturated heterocyclic group, and may be substituted, and, when $R^2$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted;
$R^3$ represents a C6-C14 aromatic hydrocarbon group or a 5- to 10-membered fully unsaturated heterocyclic group, and may be substituted, and, when $R^3$ has 2 substituents on the carbon atoms adjacent to each other on the aromatic hydrocarbon ring, the substituents may be fused together with the carbon atoms to which they bind to form a 4- to 8-membered saturated or partially unsaturated hydrocarbon ring or heterocyclic ring, which may be substituted; and
$R^4$ represents a hydrogen atom or a C1-C6 alkyl group,
provided that $X^1$ is an oxygen atom when $X^2$ represents an oxygen atom, $X^3$ represents -NH-, $X^4$ represents a hydrogen atom, $R^1$ represents $-CH_2-$, $R^2$ represents a phenyl group, $R^3$ represents a 4-methylphenyl group, and $R^4$ represents a hydrogen atom.

Fig. 1

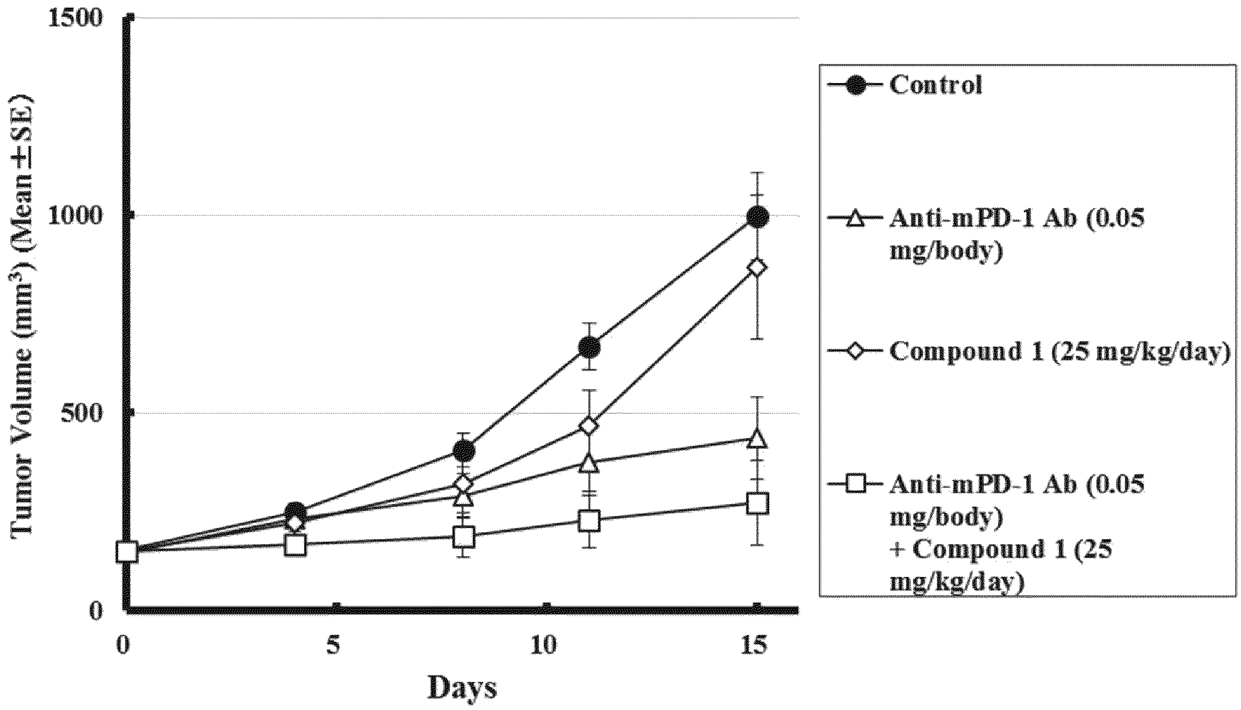
1500
1000
500
0
Tumor Volume (mm$^3$) (Mean±SE)
0
5
10
15
Days
Control
Anti-mPD-1 Ab (0.05 mg/body)
Compound 1 (25 mg/kg/day)
Anti-mPD-1 Ab (0.05 mg/body) + Compound 1 (25 mg/kg/day)

Fig. 2

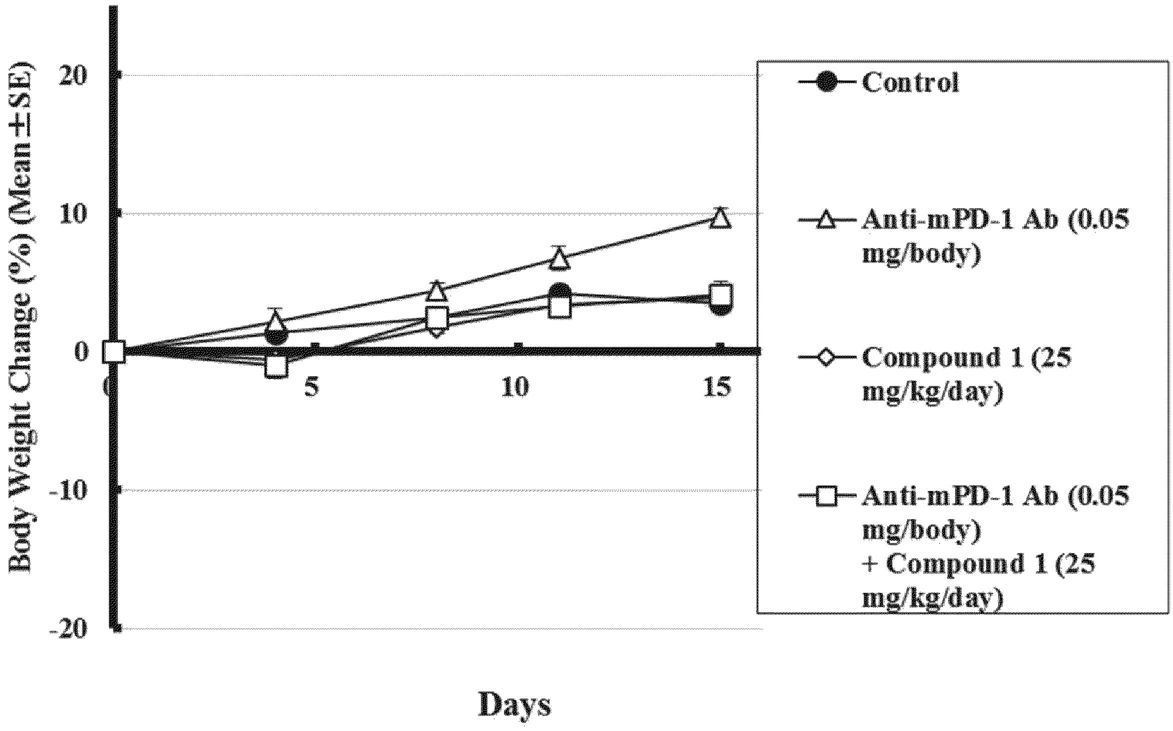
20
10
0
-10
-20
Body Weight Change (%) (Mean±SE)
0
5
10
15
Days
Control
Anti-mPD-1 Ab (0.05 mg/body)
Compound 1 (25 mg/kg/day)
Anti-mPD-1 Ab (0.05 mg/body) + Compound 1 (25 mg/kg/day)

Fig. 3

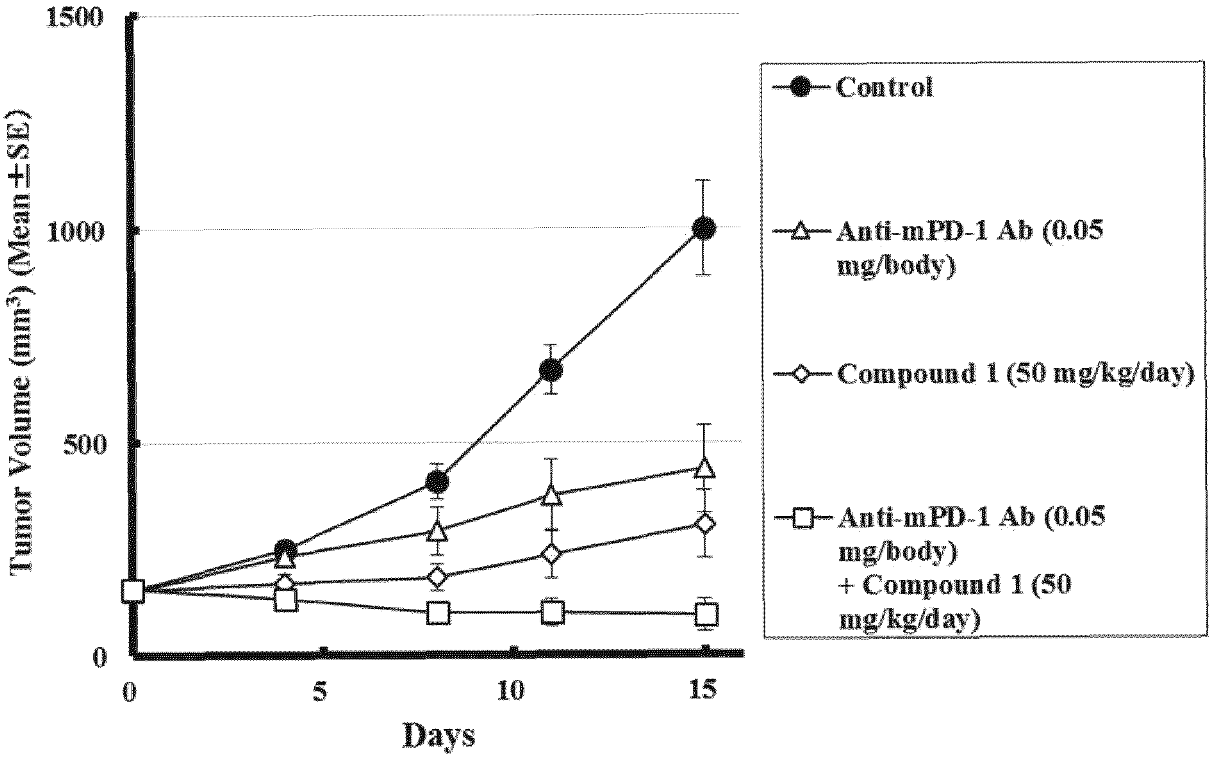
1500
1000
500
0
Tumor Volume (mm³) (Mean ± SE)
0
5
10
15
Days
Control
Anti-mPD-1 Ab (0.05 mg/body)
Compound 1 (50 mg/kg/day)
Anti-mPD-1 Ab (0.05 mg/body)
+ Compound 1 (50 mg/kg/day)

Fig. 4

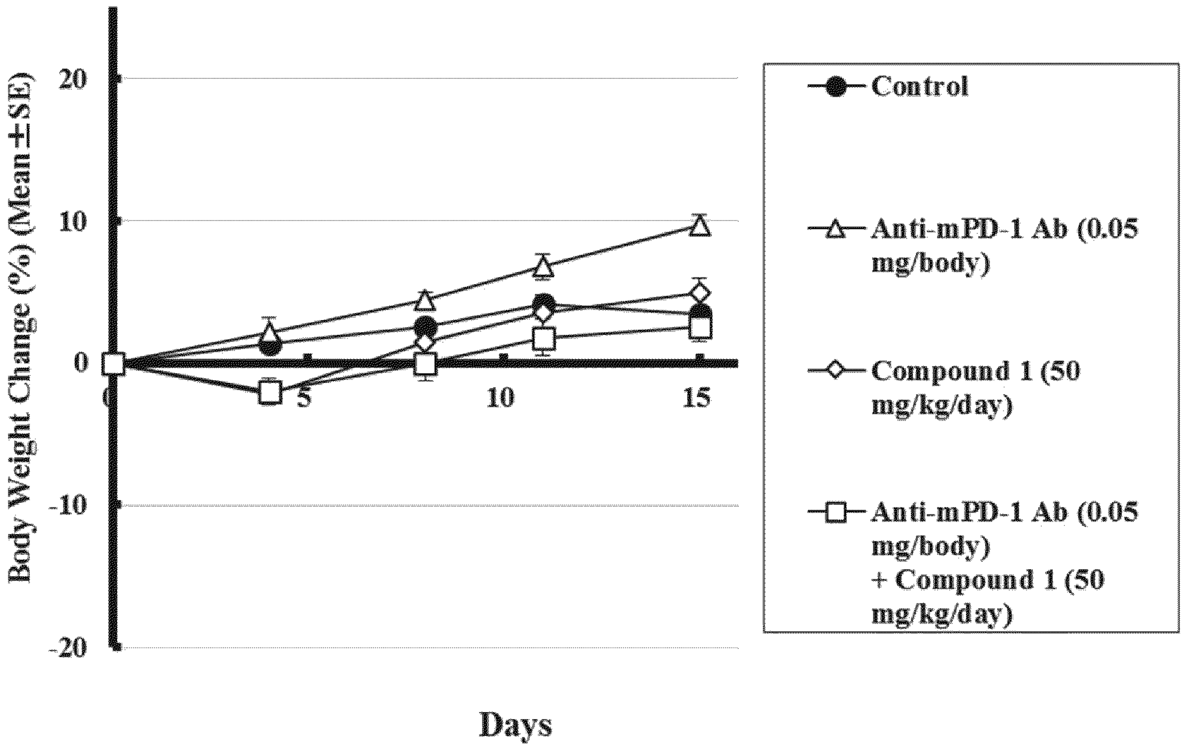
Body Weight Change (%) (Mean ± SE)
20
10
0
-10
-20
0
5
10
15
Days
Control
Anti-mPD-1 Ab (0.05 mg/body)
Compound 1 (50 mg/kg/day)
Anti-mPD-1 Ab (0.05 mg/body) + Compound 1 (50 mg/kg/day)

## INTERNATIONAL SEARCH REPORT

International application No.
PCT/JP2020/021108

A. CLASSIFICATION OF SUBJECT MATTER
A61K 39/395(2006.01)i; A61K 45/00(2006.01)i; A61P 43/00(2006.01)i; A61K 31/4245(2006.01)i
FI: A61K31/4245; A61K45/00; A61K39/395 N; A61K39/395 D; A61P43/00 121
According to International Patent Classification (IPC) or to both national classification and IPC

B. FIELDS SEARCHED
Minimum documentation searched (classification system followed by classification symbols)
A61K39/395; A61K45/00; A61P43/00; A61K31/4245

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY /MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | WO 2017/209155 A1 (TAIHO PHARMACEUTICAL CO., LTD.) 07.12.2017 (2017-12-07) each example | 12 |
| A | entire text | 1-11, 13-16 |
| A | WO 2004/004771 A1 (ONO PHARMACEUTICAL CO., LTD.) 15.01.2004 (2004-01-15) entire text | 1-16 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 July 2020 (06.07.2020) | 14 July 2020 (14.07.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/021108

| Patent Documents referred in the Report | Publication Date | Patent Family | Publication Date |
|---|---|---|---|
| WO 2017/209155 A1 | 07 Dec. 2017 | EP 3466934 A1<br>each example<br>CA 3025887 A<br>AU 2017275209 A<br>SG 11201810704W A<br>KR 10-2019-0015731 A<br>CN 109563057 A<br>MX 2018014762 A<br>BR 112018074646 A<br>TW 201800395 A | |
| WO 2004/004771 A1 | 15 Jan. 2004 | JP 2009-286795 A<br>JP 2012-236861 A<br>JP 2014-65748 A<br>JP 2015-163631 A<br>JP 2016-210817 A<br>JP 2018-39835 A<br>JP 2019-94340 A<br>US 2006/0110383 A1<br>US 2009/0297518 A1<br>US 2011/0081341 A1<br>US 2014/0314714 A1<br>US 2015/0093380 A1<br>US 2015/0197572 A1<br>US 2016/0158355 A1<br>US 2016/0158356 A1<br>US 2017/0051060 A1<br>EP 1537878 A1<br>EP 2206517 A1<br>EP 2243493 A1<br>EP 3287144 A1<br>DE 60334303 D<br>DE 10161767 T<br>AU 2003281200 A<br>AT 481985 T<br>PT 1537878 E<br>ES 2350687 T<br>DK 1537878 T<br>LU 92905 I<br>LU 92940 I<br>HU S1500066 I<br>HU S1600004 I<br>DK 2206517 T<br>ES 2654064 T<br>HU E10161767 T | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004004771 A
- WO 2017209155 A
- JP 2019100136 A


### Non-patent literature cited in the description


- *Annu. Rev. Biochem.,* 1998, vol. 67, 71-98
- *J. Biol. Chem.,* 1970, vol. 245, 5228-5233
- *Nat. Commun.,* 2014, vol. 5, 3128
- *Clin. Sci.,* 2013, vol. 124, 567-578
- *Expert. Opin. Ther. Targets,* 2013, vol. 17, 1423-1437
- *Biochem. Pharmacol.,* 2000, vol. 59, 983-991
- *Biochem. Pharmacol.,* 2009, vol. 78, 1178-1185
- *Cancer Res.,* 1994, vol. 54, 3686-3691
- *Pharmacol. Rev.,* 2005, vol. 57, 547-583
- *Future Oncol.,* 2012, vol. 8, 145-150
- *Nat. Rev. Cancer.,* 2012, vol. 12 (4), 252-64
- *N. Engl. J. Med.,* 2012, vol. 366 (26), 2443-54
- **HIROKAWA SHOTEN.** Development of Pharmaceuticals. *Molecular Design,* 1990, vol. 7, 163-198
- *Nat. Rev. Immunol.,* 2006, vol. 6, 343-357